# EUROPEAN PATENT APPLICATION

(11) **EP 4 740 841 A1**
(43) Date of publication of application: **13.05.2026**
(21) Application number: 24839694.7
(22) Date of filing: 05.07.2024
(51) Int. Cl.: A61B 5/00, A45D 44/00, A61B 5/107, A61Q 17/04, G01N 21/47, G01N 33/50, G06N 20/00

(54) **ESTIMATION METHOD, ESTIMATION DEVICE, ESTIMATION SYSTEM, AND PROGRAM**

(30) Priority: 07.07.2023 JP 2023112228; 24.11.2023 JP 2023199401; 29.03.2024 JP 2024056562
(71) Applicant: Kao Corporation, Tokyo 103-8210 (JP)
(72) Inventor: TAKEDA, Katsuya, Tokyo 103-8210 (JP); HARYUU, Yasushi, Tokyo 103-8210 (JP); OKADA, Tomonari, Tokyo 103-8210 (JP)
(74) Representative: Hoffmann Eitle
(86) International application number: PCT/JP2024/024498
(87) International publication number: WO 2025/013802

(57) **Abstract**

An estimation method includes acquiring skin condition indicators indicating skin characteristics of a user and estimating, by using the acquired skin condition indicators, a formation state of a coating film of a composition when assuming that a predetermined composition is applied to a skin of the user.

## Description

### Technical Field

The present invention relates to an estimation method of estimating a formation state of a coating film of a composition when assuming that a predetermined composition is applied to a skin of a user.

### Background Art

There is a system that diagnoses a skin or scalp from interview data and image data of keratin and/or sebum (Patent Literature 1).

### Citation List

### Patent Literature

Patent Literature 1: Japanese Patent Application Laid-open No. 2018-97899

### Disclosure of Invention

### Technical Problem

By the way, when a user applies a composition, such as a cosmetic, to the skin, ingredients contained in the cosmetic spreads on the skin uniformly if a coating film of the cosmetic is uniform, such that a sufficient effect of the cosmetic can be easily exerted. Therefore, whether or not a sufficient coating film of the cosmetic is formed is an item of high interest to users. However, according to Patent Literature 1, the state of the coating film of the applied cosmetic is not diagnosed while a skin or scalp can be diagnosed on the basis of interview data and image data.

The present invention has been made in view of such a problem. The present invention relates to an estimation method of estimating a formation state of a coating film of a composition formed on a skin of a user when the composition is applied even without applying the predetermined composition to the skin.

### Solution to Problem

The present invention relates to an estimation method including: acquiring skin condition indicators indicating skin characteristics of a user; and estimating, by using the acquired skin condition indicators and a mathematical model indicating a relationship between the skin condition indicators and formation states of a coating film of a composition when assuming that a predetermined composition is applied to a skin of the user, a formation state of a coating film of a composition when assuming that a predetermined composition is applied to the skin of the user.

Moreover, the present invention relates to an estimation apparatus including: a storage means of storing skin condition indicators indicating skin characteristics of a user and information indicating the correlation between the skin condition indicators and formation states of a coating film of a composition when assuming that a predetermined composition is applied to a skin of the user; a skin condition acquisition means of acquiring the skin condition indicators; and a coating film estimation means of referring to the storage means, estimating a formation state of a coating film of a composition when assuming that a predetermined composition is applied to the skin of the user by using the acquired skin condition indicators.

Moreover, the present invention relates to an application program that operates an estimation apparatus that estimates a formation state of a coating film of a composition when assuming that a predetermined composition is applied to a skin of a user, including: skin condition acquisition processing of acquiring skin condition indicators indicating skin characteristics of the user; and coating film estimation processing of estimating the formation state of the coating film of the composition when assuming that the predetermined composition is applied to the skin of the user by using the acquired skin condition indicators.

Moreover, the present invention relates to an estimation system including: a storage means of storing skin condition indicators indicating skin characteristics of a user and information indicating the correlation between the skin condition indicators and formation states of a coating film of a composition when assuming that a predetermined composition is applied to a skin of the user; a skin condition acquisition means of acquiring the skin condition indicators; and a coating film estimation means of referring to the storage means, estimating a formation state of a coating film of a composition when assuming that a predetermined composition is applied to the skin of the user by using the acquired skin condition indicators.

### Advantageous Effects of Invention

In accordance with the method provided in accordance with the present invention, it is possible to estimate a formation state of a coating film of a composition when assuming that the predetermined composition is applied to the skin of the user.

### Brief Description of Drawings

[Fig. 1] An image view when acquiring skin condition indicators of Embodiment 1.
[Fig. 2-1] An image view when acquiring a reflectance of a skin to which a predetermined cosmetic has been applied in Embodiment 1 or 2.
[Fig. 2-2] (a) is a facial image of a bare skin captured in Fig. 2-1 and (b) is a facial image captured in Fig. 2-1, to which the cosmetic has been applied.
[Fig. 3] (a) is an image view in a case where a bare skin was irradiated with light with a wavelength of 320 nm in a UVB region and (b) is an image view in a case where a coated skin to which the cosmetic has been applied is irradiated with light with a wavelength of 320 nm in the UVB region.
[Fig. 4] (a) shows results of predicting whether it is sorted into a cluster whose level of the formation state of the coating film of the cosmetic is lower or a cluster whose level is higher by using a coating film estimation mathematical model 1 and (b) is a diagram showing a matching degree of a cluster predicted by using the coating film estimation mathematical model 1 and an actual cluster as an accuracy rate.
[Fig. 5] An image view when acquiring a skin image (bare skin image) in Embodiment 2 or 3.
[Fig. 6] (a) shows results of predicting whether it is sorted into a cluster whose level of the formation state of the coating film of the cosmetic is lower or a cluster whose level is higher by using a coating film estimation mathematical model 2 and (b) is a diagram showing the matching degree of a cluster predicted by using the coating film estimation mathematical model 2 and an actual cluster as an accuracy rate.
[Fig. 7] An image view when acquiring an evaluation value of an overall evaluation of a skin to which a predetermined cosmetic has been applied in Embodiment 3.
[Fig. 8] (a) shows results of predicting whether it is sorted into a cluster whose level of the formation state of the coating film of the cosmetic is lower or a cluster whose level is higher by using a coating film estimation mathematical model 3 and (b) is a diagram showing the matching degree of a cluster predicted by using the coating film estimation mathematical model 3 and an actual cluster as an accuracy rate.
[Fig. 9] A diagram when performing estimations with respect to an effect claimed for the cosmetics when assuming that Cosmetics 1 to 3 have been applied.
[Fig. 10-1] A block diagram of an estimation apparatus.
[Fig. 10-2] A block diagram of the estimation apparatus.
[Fig. 11-1] A block diagram of an estimation system.
[Fig. 11-2] A block diagram of the estimation system. [Fig. 12] (a-1) to (a-3) are display format examples of an skin information classification image, (b-1) to (b-3) are display format examples of a composition classification image, (c-1) is a display format example of the skin information classification image and the composition classification image, and (d-1) and (d-2) are display format examples of the skin information classification image and the composition classification image.
[Fig. 13] A diagram showing results of comparison tests of a UV protection effect of cosmetics C to E.
[Fig. 14] A diagram showing results of other comparison tests of the UV protection effects of the cosmetics C to E.
[Fig. 15] A flowchart showing a flow of presentation processing of skin type characteristic information and cosmetic score information based on a skin image by the estimation apparatus or the estimation system.
[Fig. 16] A diagram showing an example of the skin type characteristic information which is generated and stored in the estimation apparatus or the estimation system.
[Fig. 17] A diagram showing an example of score information related to the UV protection effect of the cosmetic for each skin type, which is generated and stored in the estimation apparatus or the estimation system.
[Fig. 18] A diagram showing an example of the skin type characteristic information and the cosmetic score information, which the estimation apparatus or the estimation system causes an information processing terminal of a user to display.

### Mode(s) for Carrying Out the Invention

Hereinafter, an example of a favorable embodiment of the present invention will be described with reference to the drawings. It should be noted that the drawings of the present embodiment are all for describing technological concepts, configurations, and operations of the present invention, and not limit its configurations to specific ones. Moreover, in all drawings, similar components are denoted by similar reference signs and duplicate descriptions are omitted as appropriate.

The outline of an estimation method in the present embodiment (hereinafter, it may be referred to as this method) will be described.

The estimation method according to the present embodiment is a method of acquiring skin condition indicators indicating skin characteristics of a user and estimating, by using the acquired skin condition indicators and a mathematical model indicating a relationship between the skin condition indicators and formation states of a coating film of a composition when assuming that a predetermined composition is applied to a skin of the user, a formation state of a coating film of a composition when assuming that a predetermined composition is applied to a skin of the user.

The "skin condition indicators indicating the skin characteristics of the user" are indicators indicating skin characteristics of the skin of the user to which a predetermined composition, a predetermined cosmetic, is not applied, a skin after face washing, a so-called bare skin. Here, the "skin characteristics" are skin properties and mean a skin type, for example, a dry skin, normal skin, oily skin, or mixed skin. The indicators indicating these skin characteristics are the "skin condition indicators." The indicators are favorably indicators in terms of physical properties of the skin, such as stratum corneum water content, transepidermal water loss, skin viscoelasticity, softness such as skin elasticity, contact angle, tone, melanin content, melanin density, skin temperature, moisture content, water-oil balance, surface shape, skin texture, wrinkles, acne, and moles. The indicators also include skin impressions indicating conditions of the skin, for example, skin age, makeup intensity, masculinity/femininity, degree of makeup smudging, and powderiness. It should be noted that skin types can be classified as those other than those generally known skin types as appropriate on the basis of the skin condition indicators. Moreover, the skin type classification is based on predetermined elements (e.g., three elements, "skin texture," "water-oil balance," and "softness") included in the skin condition indicators. Any method can be employed for the skin type classification. Conditions for classifying as respective skin types may be determined in advance. For example, the conditions include that a value of a predetermined element of the values of the three elements is greater than or equal to a predetermined value, that all elements are greater than or equal to a predetermined value, and that a value of a predetermined element is greater than or equal to a predetermined position and values of the other elements are less than or equal to the predetermined value. Then, the classification may be performed on the basis of the conditions. Moreover, for example, five clusters may be generated from, for example, the values of the three elements, "skin texture," "water-oil balance," and "softness," by cluster analysis and skin types may be made to respectively correspond to the generated clusters. Then, which cluster the skin belongs to may be analyzed from the values of the three elements included in the skin condition indicators of the user and the skin may be classified as the skin type corresponding to the corresponding cluster. It should be noted that those three elements are exemplary, and the types of elements and the number of elements are not limited thereto.

The "skin of the user" means a soft tissue of a superficial layer of a living body. It can be any body site, such as a face, arm, or hand.

The "predetermined composition" is a composition intended to be applied to the skin. It broadly includes cosmetics, quasi-drugs, and medications called, for example, lotions, toners, creams, and milky lotions. Specific examples of the "predetermined composition" include: skin care cosmetics such as lotions, toners, milky lotions, creams, serums, massage masks, lip balms; makeup cosmetics such as foundations, makeup bases, liquid foundations, oil-based foundations, powder foundations, concealers, control colors, eye shadows, cheek powders, lipsticks, lip glosses, lip liners, and decollete cosmetics; UV protective cosmetics such as sun milk, sunscreen gels, and sun cream; and bath cosmetics such as bath oil and bath additive and ointments containing antiseptic ingredients, anti-itch ingredients, inflammation reducing ingredients, and the like, though not particularly limited thereto.

The "coating film of the composition" is a coating film formed on a skin by applying a predetermined composition on the skin. As will be described later in detail, the formation state of the coating film of the composition differs depending on conditions of the skin to be coated even when the same amount of the same composition is applied. Moreover, the formation state of the coating film of the composition differs depending on a type of (ingredient) of the composition and an application amount even when the composition is applied to the same skin.

The "formation state of the coating film" is how the coating film of the composition is formed on the skin. The "formation state of the coating film" is evaluated in terms of the thickness of the formed coating film, unevenness of the thickness of the formed coating film, and uniformity of the formed coating film. Examples of the "formation state of the coating film" includes a state in which the coating film of the composition is formed with no color unevenness in a case where the composition to be applied includes a color, a state in which the coating film of the composition is formed in a state in which particles are uniform in a case where the composition to be applied contains the particles, a state in which UV rays are absorbed regardless of a position where the coating film of the composition is formed in a case where the composition to be applied contains UV absorbing agents, and a state in which UV rays are reflected regardless of a position where the coating film of the composition is formed in a case where the composition to be applied contains UV scattering agents.

The "estimating" is estimating the formation state of the coating film of the composition formed when the predetermined composition is applied without applying the predetermined composition on the skin of the user. The estimation result may be in any form, such as a numeric value output, a graph-based output, or a simulated image output.

The "skin information classification image" is any image indicating a skin type classified on the basis of the skin condition indicators.

The "composition classification image" is any image indicating composition classification performed on the basis of characteristics of the composition. The "characteristics of the composition" is not particularly limited as long as they are factors associated with the formation state of the coating film of the composition. Examples of the "characteristics of the composition" include a formulation type of the composition, physical properties such as wettability, viscosity, and color.

The skin information classification image and the composition classification image are each generated by shape, pattern or color, or shading, or a combination thereof. The estimation results can be output by combining these images, such as by overlaying them.

As a predetermined composition applied to a skin of a subject when generating a coating film mathematical model prepared in the present embodiment, a cosmetic to which at least either ingredients (hereinafter, also referred to as "UV absorbing agents") that absorb light with wavelengths in the ultraviolet region or ingredients (hereinafter, also referred to as "UV scattering agents") that scatter light with wavelengths in the ultraviolet region have been added was used. Here, the "cosmetic to which ... have been added" includes not only a cosmetic to which at least either ingredients, UV absorbing agents or UV scattering agents, have been added (containing these ingredients), but also a cosmetic obtained by adding the ingredients to a cosmetic not containing these ingredients at a predetermined rate. Accordingly, it is also possible to estimate a formation state of the coating film of the cosmetic when assuming that a cosmetic not containing the ingredients that absorb and/or scatter light with wavelengths in the ultraviolet region is applied to the skin of the user.

In the present embodiment, for example, a cosmetic obtained by adding the UV absorbing agents and/or the UV scattering agents to a W/O cosmetic (water-in-oil cosmetic) or an O/W cosmetic (oil-in-water cosmetic) was used. Therefore, it is possible to estimate UV protection properties of the cosmetic by using an estimation result of estimating the formation state of the coating film of the cosmetic formed on the skin when assuming that the cosmetic to which the UV absorbing agents and/or the UV scattering agents have been added is applied to the skin of the user. It should be noted that when generating a coating film mathematical model, it may be only a cosmetic to which only the UV absorbing agents have been added, a cosmetic to which only the UV scattering agents have been added, or a cosmetic to which both the UV scattering agents and the UV absorbing agents have been added. Also in these cases, it is possible to estimate the UV protection properties of the cosmetic on the basis of the estimation result.

In some cosmetic to which the UV absorbing agents and/or the UV scattering agents have been added, an indicator indicating the UV protection properties is specified (published UV protection value published in advance). As the indicator indicating the UV protection properties, Sun Protection Factor (SPF) related to UV rays in the UVB region is most widely known, and the UV protection effect is displayed as an SPF value (e.g., "SPF30," etc.). Moreover, regarding UV rays in the UVA region, PFA (Protection Factor of UVA) or UVAPF (UVA Protection factor of Sun Protection Factor product) is used. When evaluating the UV protection properties of the cosmetic, an evaluation may be made with respect to the specified indicator indicating the UV protection properties.

The predetermined composition with respect to which the formation state of the coating film is estimated in the present embodiment is a cosmetic to which at least either ingredients that absorb light with wavelengths in the ultraviolet region or ingredients that scatter light with wavelengths in the ultraviolet region have been added. The estimated formation state of the coating film of the composition will be described as the formation state of the coating film of the cosmetic. Moreover, estimating whether a function of the cosmetic (e.g., whether or not a claimed UV protection function can be expected) can be exerted on the basis of the estimated formation state of the coating film of the cosmetic will be described.

### <Embodiment 1>

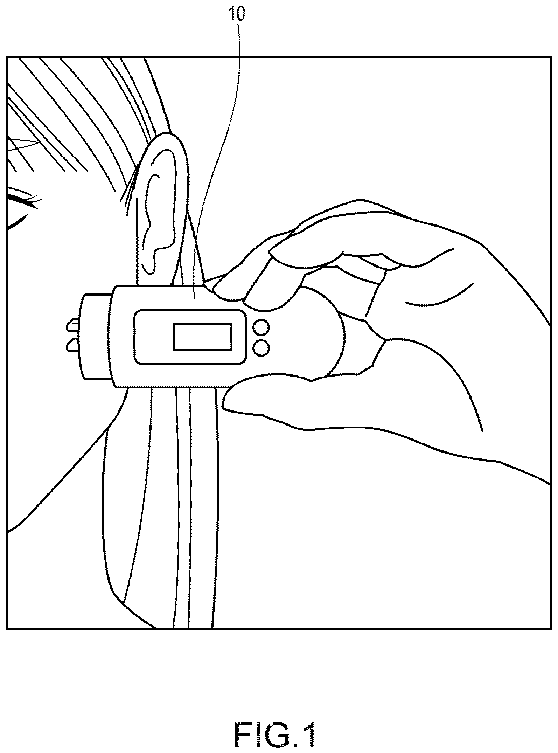

As shown in Fig. 1, Embodiment 1 is a method of measuring (acquiring) skin condition indicators with a predetermined measurement instrument 10 with respect to a skin (bare skin) of a user and estimating, on the basis of the measured skin condition indicators, a formation state of a coating film formed on the skin by the cosmetic when assuming that a predetermined cosmetic is applied. The formation state of the coating film of the cosmetic is estimated on the basis of the measured skin condition indicators and a mathematical model generated in advance (mathematical model used in the coating film estimation according to Embodiment 1 will be referred to as a "coating film estimation mathematical model 1"). In Embodiment 1, the formation state of the coating film formed on the skin when assuming that a cosmetic A (SPF50) and a cosmetic B (SPF50+) is applied is estimated as the predetermined cosmetic.

The skin condition indicators acquired in Embodiment 1 are physical property values indicating skin conditions which are measured with the predetermined measurement instrument 10 with respect to the skin of the user. Examples of the physical property values include stratum corneum water content, transepidermal water loss, skin viscoelasticity, skin elasticity, and contact angle.

Fig. 1 shows measuring a transepidermal water loss by putting a contact element in contact with the skin. The skin condition indicators to be acquired is shown below.
- Stratum corneum water content: a value measured by a skin hydration measurement instrument (Corneometer, which is manufactured by Courage+Khazaka) is used.
- Transepidermal water loss: a value measured by a transepidermal water loss measurement instrument (Tewameter, which is manufactured by Courage+Khazaka) is used.
- Skin viscoelasticity: a value measured by a skin viscoelasticity measurement instrument (Cutometer, which is manufactured by Courage+Khazaka) is used.
- Skin elasticity: a value measured by a skin viscoelasticity measurement instrument (Cutometer, which is manufactured by Courage+Khazaka) is used.
- Contact angle: a value measured by a contact angle measurement instrument (portable contact angle meter PCA-11, which is manufactured by Kyowa Interface Science Co., Ltd) is used.

It should be noted that those measurement instruments are exemplary, and values measured by other measurement instruments or values calculated by capturing an image of the skin and processing the captured image may be used.

In this manner, in Embodiment 1, the skin condition indicators include an evaluation item of at least one of the stratum corneum water content, transepidermal water loss, skin viscoelasticity, skin elasticity, and contact angle, which are measured by the predetermined measurement instrument.

Next, the coating film estimation mathematical model 1 will be described.

Training data used for generating the coating film estimation mathematical model 1 and conditions thereof are as follows.

| | |
|---|---|
| - Predetermined composition 20: | cosmetic A (SPF50, formulation type: W/O) |
| | cosmetic B (SPF50+, formulation type: W/O) |
| - Application area | : 4 cm × 4 cm |
| - Application amount | : 16 mg for 4 cm × 4 cm |
| - Target subject | : foreheads or cheeks of 19 |
| | males and females for the cosmetic A |
| | foreheads or cheeks of 20 males |
| and females for the cosmetic B | |
| - Used camera | : UV camera 30 |
| - Application method | : a trained examiner applies them uniformly. |
| - Light source | : MAX-303, which is manufactured by Asahi Spectra Co., Ltd. |
| - Bandpass filter (320 nm ±5 nm) | |
| - Polarization plate | |

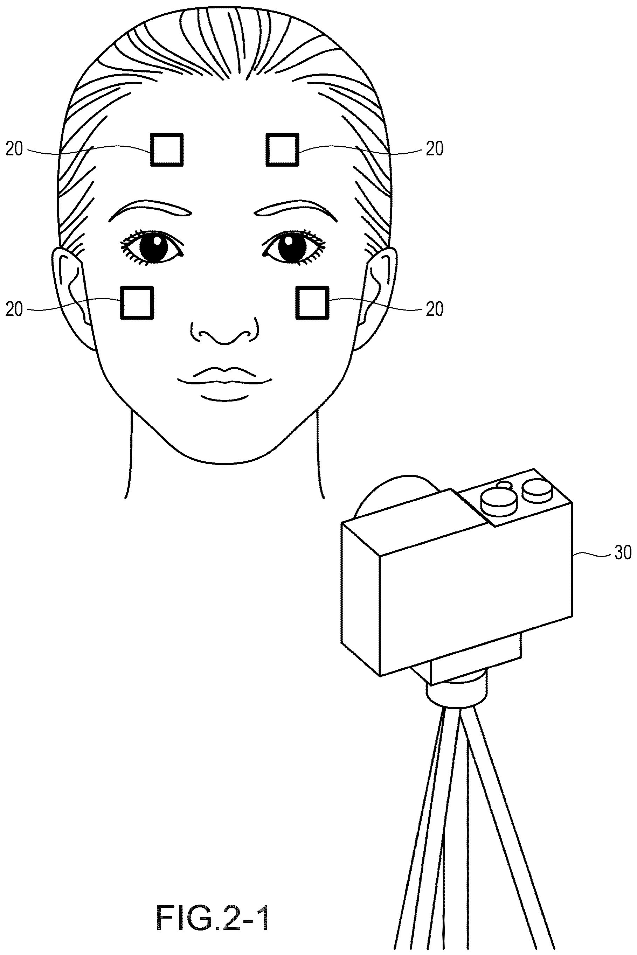

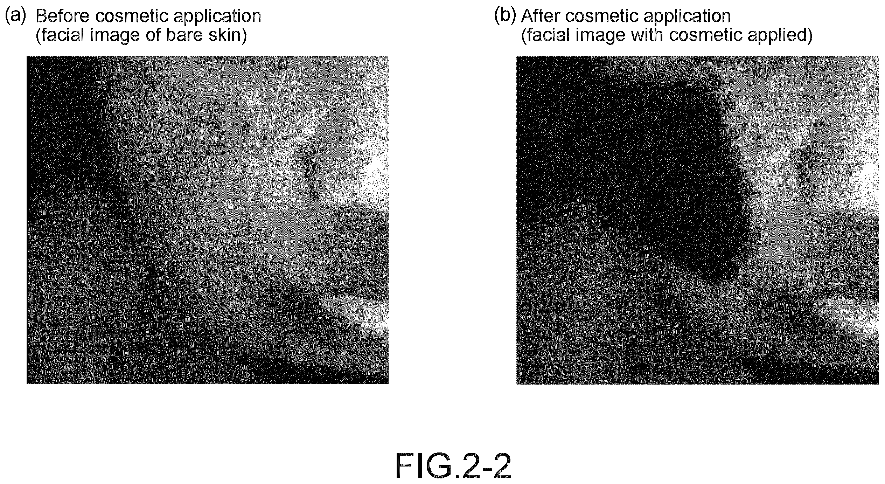

As shown in Fig. 2-1, the predetermined composition 20 is applied to the skin of the subject, the skin of the subject is imaged with the UV camera 30 under UV irradiation, and the thus captured image data (UV skin image) is used. The coating film estimation mathematical model 1 is generated from the captured image data by the following procedure.

### 1) Calculate a UV reflectance of the coating film of each cosmetic formed on the skin

A UV reflectance of the coating film (referred to as a "UV reflectance A") formed on the skin by the cosmetic A in a state in which the predetermined composition 20 (cosmetic A) is applied to the skin (skin 15) of the subject and a UV reflectance of the coating film (referred to as a "UV reflectance B") formed on the skin by the cosmetic B in a state in which the predetermined composition 20 (cosmetic B) is applied to the skin of the subject are calculated. The calculation method is shown below.

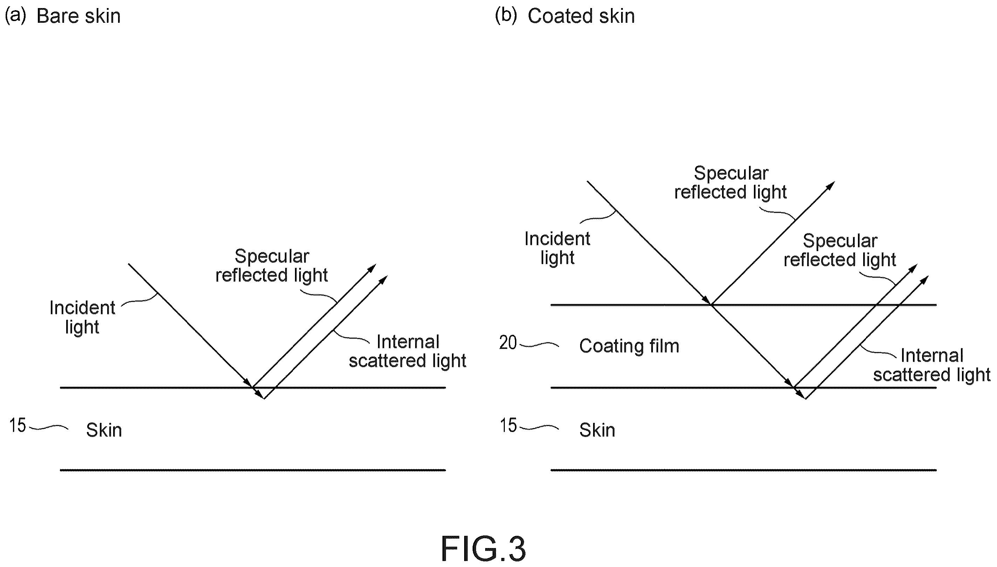

Fig. 3 (a) shows a case where the skin (bare skin) of the subject is irradiated with UV rays (hereinafter, "UV light") with a wavelength of 320 nm included in the UVB region. "Incident light" in the figure is the irradiated UV light. "Positive reflection light" in the figure is UV light reflected on the surface of the skin. "Internal scattered light" in the figure is UV light that is not reflected on the surface of the skin, enters the skin, spreads inside the skin, and goes out of the skin the UV light. The intensity of the internal scattered light, from which the positive reflection light is removed, is acquired. This is handled as internal scattered light intensity of the bare skin.

Fig. 3 (b) shows a case where the predetermined composition 20 is applied to the skin of the subject and is irradiated with UV light. "Incident light" in the figure is the irradiated UV light. "Positive reflection light" in the figure is UV light reflected on the surface of the predetermined composition 20 and UV light reflected on the surface of the skin. "Internal scattered light" in the figure is UV light that is not reflected on the surface of the predetermined composition 20 and the surface of the skin enters the skin, spreads inside the skin, and goes out of the skin the UV light. The intensity of the internal scattered light, from which the positive reflection light in a case where the cosmetic A is applied as the predetermined composition 20 is removed, and the intensity of the internal scattered light, from which the positive reflection light in a case where the cosmetic B is applied as the predetermined composition 20 is removed, are acquired. These will be respectively referred to as internal scattered light intensity A of the coated skin to which the cosmetic A is applied and internal scattered light intensity B of the coated skin to which the cosmetic B is applied.

Then, by dividing the internal scattered light intensity of the coated skin when the predetermined composition 20 is applied by the internal scattered light intensity of the bare skin, a UV reflectance of the composition present in the site to which the predetermined composition 20 is applied is calculated (the internal scattered light intensity A of the coated skin to which cosmetic A is applied/the internal scattered light intensity of the bare skin = the UV reflectance of the coating film (UV reflectance A) formed by the cosmetic A present in the site to which cosmetic A is applied, the internal scattered light intensity of the coated skin B to which the cosmetic B is applied/the internal scattered light intensity of the bare skin = the UV reflectance of the coating film (UV reflectance B) formed by the cosmetic B present in the site to which the cosmetic B is applied).

In this manner, the positive reflection light is removed and the UV reflectance is calculated with the internal scattered light intensity. Accordingly, it is possible to estimate an application state of the coating film (formation state of the coating film) formed on the skin with high accuracy.

Here, the internal scattered intensity can be measured by mounting polarization plates on the light source and the UV camera, thereby irradiating the skin to which the cosmetic A (or the cosmetic B) is applied with UV light, removing the positive reflection light, and capturing an image of the reflected internal scattered light with the UV camera 30.

It should be noted that in the present embodiment, the UV reflectance with a wavelength of 320 nm is calculated in view of the fact that the wavelength range of the UVB region is 320 to 280 nm, though the wavelength range is not limited thereto. For example, in a case of estimating a formation state of the coating film formed on the skin by the cosmetic and estimating a UV protection effect of the UVA region from the estimation result, the reflectance of UV rays may be calculated by using UV rays with any one of wavelengths of 380 to 320 nm, e.g., a wavelength of 350 nm.

### 2) Sort into two clusters in accordance with the UV reflectance

The calculated UV reflectances of the coating films formed on the skin by the respective cosmetics are arranged in order of the UV reflectance and the respective subjects are sorted into two clusters, that with a higher UV reflectance (50%) and that with a lower UV reflectance (50%). The cluster whose UV reflectance is higher will be referred to as a "cluster whose level of the formation state of the coating film of the cosmetic is lower" and the cluster whose UV reflectance is lower will be referred to as a "cluster whose level of the formation state of the coating film of the cosmetic is higher." In general, a thinner coating film of the cosmetic is formed when the UV reflectance is higher while a thicker coating film of the cosmetic is formed when the UV reflectance is lower. Regarding this difference, in a case where the cosmetic-applied area and the cosmetic application amount are constant as in the present embodiment, for example, if the skin surface to which the cosmetic is applied has recess portions, such as wrinkles and acne scars, a thinner coating film of the cosmetic is formed on the skin because the cosmetic is applied to fill these recess portions. The conditions of the skin to be coated affect the formation state of the coating film of the cosmetic in this manner. Therefore, the UV reflectances of the coating films formed on the skins by the respective cosmetics, which are calculated by the subjects, differ (can be ordered). It should be noted that the skin surface shape has been described as a factor that affects the formation state of the coating film of the cosmetic, though this is merely an example. Differences of other skin conditions, skin physical property values, are factors that affect the formation state of the coating film of the cosmetic.

### 3) Measure skin condition indicators of the subject

The above-mentioned skin condition indicators (stratum corneum water content, transepidermal water loss, skin viscoelasticity, skin elasticity, and contact angle) are measured with the predetermined measurement instrument with respect to the above-mentioned subject.

### 4) Generate training data for use in machine learning

Training data for generating the coating film estimation mathematical model 1 is generated by using the result of sorting into the two clusters in 2) and the measurement result in 3).

### 5) Generate a coating film estimation mathematical model 1

For each evaluation value, standardization processing is performed, optimal one is selected from a plurality of classification models (e.g., naive bayes (NB), logistic regression (LR), linear discriminant analysis (LDA), random forest (RF), gradient boosting (GB), support vector machine (SVM), Gaussian process (GP)) and set as a prediction model, and whether it is sorted into a cluster whose level of the formation state of the coating film of the cosmetic is lower or a cluster whose level is higher is predicted by using this prediction model. A matching degree of the predicted cluster and the actual cluster is calculated as an accuracy rate. The generalization performance of the classification model is evaluated by leave-one-out cross-validation (LOOCV) and a calculation model having the highest accuracy rate is set as an optimal model: coating film estimation mathematical model 1. It should be noted that in the present embodiment, a classification model GB was used for the coating film estimation mathematical model 1 of the cosmetic A and a classification model LDA was used for the coating film estimation mathematical model 1 of the cosmetic B.

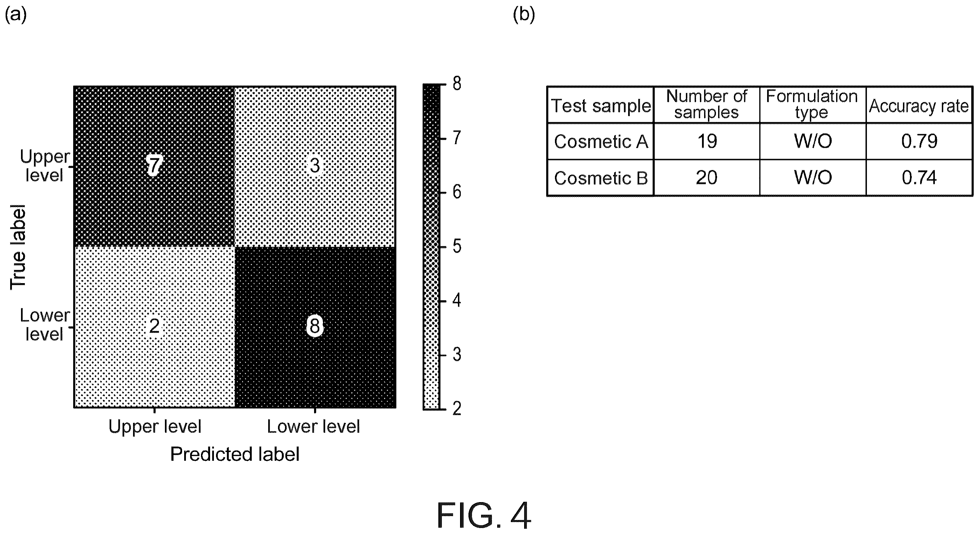

Fig. 4 (a) shows results of predicting whether it is sorted into the cluster whose level of the formation state of the coating film formed on the skin by the cosmetic is lower or the cluster whose level is higher by using the coating film estimation mathematical model 1 and actual results. Fig. 4 (b) shows results of calculating a matching degree of the cluster predicted and the actual cluster as an accuracy rate by using the coating film estimation mathematical model 1.

It can be seen from Fig. 4 (a) and (b), a high accuracy rate is obtained in a case of predicting the formation state of the coating film formed on the skin by the cosmetic by using the coating film estimation mathematical model 1.

Therefore, the formation state of the coating film of the cosmetic can be estimated by using the skin condition indicators of the user that are acquired by the predetermined measurement instrument and the coating film estimation mathematical model 1 without applying the predetermined cosmetic to the skin. Therefore, it is possible to estimate a formation state of the coating film of the cosmetic formed on the skin (whether the level of the formation state of the coating film formed on the skin by the cosmetic is high or low) when the predetermined cosmetic is applied to the skin of the user even without applying the predetermined cosmetic (cosmetic to which the UV absorbing agents and/or the UV scattering agents have been added) to the skin. Accordingly, it is also possible to evaluate to which extent an effect based on a function of the predetermined cosmetic (e.g., a UV protection function in a case of the cosmetic to which the UV absorbing agents and/or the UV scattering agents have been added) can be expected.

As described above, Embodiment 1 includes generating a mathematical model indicating a relationship between the skin condition indicators and UV reflectances of the coating film of the cosmetic (coating film estimation mathematical model 1) by using skin condition indicators (e.g., stratum corneum water content, transepidermal water loss, skin viscoelasticity, skin elasticity, and contact angle by the measurement instrument 10) of each of a plurality of subjects (e.g., 19 to 20 males and females) and the UV reflectance of the coating film formed on the skin by the cosmetic, which is calculated from UV skin images (e.g., the images shown in Fig. 2-2 captured by the imaging device shown in Fig. 2-1) of each of the plurality of subjects. It is possible to estimate a formation state of the coating film formed on the skin by the cosmetic when assuming that the cosmetic is applied to the skin of the user by using the generated coating film estimation mathematical model 1 and the skin condition indicators of the user measured by the measurement instrument 10 (e.g., stratum corneum water content, transepidermal water loss, skin viscoelasticity, skin elasticity, and contact angle). It is possible to estimate and evaluate the function exerted by the cosmetic.

Moreover, the formation state of the coating film formed on the skin by the cosmetic is estimated by using the reflectance of the UV light with a wavelength of 320 nm in the wavelength range of UVB, so it is possible to directly estimate and evaluate the function of the cosmetic (the UV protection function in the UVB region for a cosmetic with a UV protection function in the UVB region) on the basis of the formation state of the coating film formed on the skin by the cosmetic. Specifically, for example, in a case of the cosmetic A (SPF50), the UV reflectance of each of the plurality of subjects (19 subjects) is calculated and a standard value of the calculated UV reflectances is determined. The "standard value" in the present embodiment is a UV reflectance located at the center when the UV reflectances are arranged in order of the UV reflectance. It has been described in "2) Sort into two clusters in accordance with the UV reflectance." Then, the following estimations can be made. Specifically, in a case where the formation state (UV reflectance) of the coating film formed on the skin by the cosmetic of the subject, which is estimated by the above-mentioned method, is equal to the standard value, the effect claimed for the cosmetic A (equivalent to SPF50) can be expected. In a case where it is greater than the standard value, the effect claimed for the cosmetic A (equivalent to SPF50) can be expected to be sufficient. In a case where it is less than the standard value, the effect claimed for the cosmetic A (equivalent to SPF50) cannot be expected to be sufficient. Thus, the user can judge whether or not that cosmetic is suitable to the user's skin on the basis of the estimated formation state of the coating film formed on the skin by the cosmetic.

In general, the formation state of the coating film formed on the skin by the cosmetic changes over time after the predetermined cosmetic is applied to the skin. It is caused by various factors such as external factors such as humidity, temperature, and winds, factors due to skin characteristics of a subject (user), and physical constitution of the subject (user) (whether or not the user sweats easily, whether or not the skin surface moves frequently (as to the face, whether or not the facial expression changes frequently)).

In view of this, the coating film estimation mathematical model 1 may be configured to estimate a formation state (hereinafter, referred to as a "first formation state") of the coating film formed on the skin by the cosmetic in a first time zone after the predetermined cosmetic is applied to the skin of the user and a formation state (hereinafter, referred to as a "second formation state") of the coating film formed on the skin by the cosmetic in a second time zone later than the first time zone.

A method of estimating the first formation state and the second formation state will be described.

Here, the first time zone is, for example, 1 minute later after application and the second time zone is, for example, 8 hours later after application. It is only sufficient to generate coating film estimation mathematical models 1 in the respective times. That is, it is only sufficient that the above-mentioned process of "1) Calculate a UV reflectance of the coating film of each cosmetic formed on the skin" is performed, UV reflectances of each cosmetic are calculated when 1 minute has elapsed since the predetermined cosmetic was applied to the skins of the plurality of subjects and when 8 hours have elapsed since the predetermined cosmetic was applied to the skins of the plurality of subjects, respectively, the above-mentioned processes of "2) Sort into two clusters in accordance with the UV reflectance" to "5) Generate a coating film estimation mathematical model 1" are performed, and a coating film estimation mathematical model 1 to estimate the formation state (first formation state) of the coating film formed on the skin by the cosmetic 1 minute later after application and a coating film estimation mathematical model 1 to estimate the formation state (second formation state) of the coating film formed on the skin by the cosmetic 8 hours later after application are configured.

In this manner, the coating film estimation mathematical model 1 to estimate the formation state (first formation state) of the coating film formed on the skin by the cosmetic 1 minute later after application and the coating film estimation mathematical model 1 to estimate the formation state (second formation state) of the coating film formed on the skin by the cosmetic 8 hours later after application are generated, and the two generated coating film estimation mathematical models 1 and the skin condition indicators of the user that are acquired by the predetermined measurement instrument are used. Accordingly, it is possible to estimate a formation state of the coating film formed on the skin by the cosmetic after the predetermined time since application without applying the predetermined cosmetic to the skin of the user and even when a predetermined time (in the present embodiment, the first time zone: 1 minute or the second time zone: 8 hours) has not elapsed since application.

In this manner, it is possible to estimate the formation state (first formation state) of the coating film formed on the skin by the composition in the first time zone (e.g., 1 minute after application) after application in which the predetermined composition (e.g., cosmetic A, cosmetic B) is applied and the formation state (second formation state) of the coating film formed on the skin by the composition in the second time zone later than the first time zone (e.g., 8 hours after application). It should be noted that it is not necessarily necessary to estimate both the first formation state and the second formation state, and only either one of them may be estimated depending on a purpose. Moreover, it is also possible to increase the number of time zones, for example, a third time zone, a fourth time zone, and so on.

Moreover, it is possible to estimate each of an effect that can be expected in the first time zone and an effect that can be expected in the second time zone with respect to the effect claimed for the applied composition by using the first formation state and the second formation state.

As described above, the predetermined cosmetic is the cosmetic (e.g., cosmetic A, cosmetic B) to which the UV absorbing agents and/or the UV scattering agents have been added, and it is possible to estimate the first formation state and the second formation state and estimate and evaluate the function exerted by the cosmetic on the basis of the estimated first formation state and second formation state (evaluate to which extent the effect can be expected with respect to the function of the cosmetic).

Therefore, for example, if the user wishes to estimate and evaluate the UV protection effect in a case where the user applies the cosmetic before go-out and enjoys activity in a circumstance where the user is easily exposed to UV rays 8 hours later, it is only sufficient to estimate the formation state of the coating film formed on the skin by the cosmetic in the second time zone. Moreover, for example, if the user wishes to estimate the UV protection effect in a case where the user applies the cosmetic before go-out and enjoys activity continuously for 8 hours in a circumstance where the user is easily exposed to UV rays, it is possible to estimate both formation states of the coating films formed on the skin by the cosmetic in the first time zone and the second time zone and estimate and evaluate it by using both. In this manner, it is possible to use the coating film estimation mathematical model 1 depending on each purpose.

### <Embodiment 2>

Embodiment 2 is a method of estimating a formation state of a coating film formed on a skin when assuming that a predetermined composition is applied on the basis of skin condition indicators generated from an image obtained by imaging a skin (bare skin) of a user by using a mathematical model and a mathematical model indicating a relationship between the skin condition indicators and the UV reflectance when the predetermined composition is applied to the skin.

In the present embodiment, the mathematical model for use for generating the skin condition indicators will be referred to as a "skin condition indicator mathematical model." Moreover, the mathematical model indicating the relationship between the skin condition indicators acquired in Embodiment 2 and the UV reflectance of the coating film of the composition when assuming that the predetermined composition is applied to the skin will be referred to as a "coating film estimation mathematical model **2."** In Embodiment 2, the formation state of the coating film formed on the skin by the composition is estimated by using the "skin condition indicator mathematical model" and the "coating film estimation mathematical model 2" without applying the composition to the skin of the user. In Embodiment 2, as the predetermined composition, the formation state of the coating film formed on the skin when assuming that the cosmetic C (SPF50+, formulation type: O/W) is applied is estimated by using two types of mathematical models, the "skin condition indicator mathematical model" and the "coating film estimation mathematical model 2."

The skin condition indicator mathematical model and the coating film estimation mathematical model 2 will be described.

The "skin condition indicator mathematical model" is a learned discrimination model learned on the basis of a plurality of pieces of training data and is a discrimination model learned to be capable of acquiring at least seven skin condition indicators as follows.
- Overall evaluation (overall skin condition level)
- Skin tone (skin brightness)
- Makeup intensity (whether or not the skin looks well made up)
- Skin age (indicator indicating a skin aging degree)
- Masculinity/femininity (whether the skin texture is closer to that of a male or female)
- Makeup smudge level (indicator indicating the extent to which makeup smudges)
- Powderiness (indicator indicating a silky smooth feel of the skin)

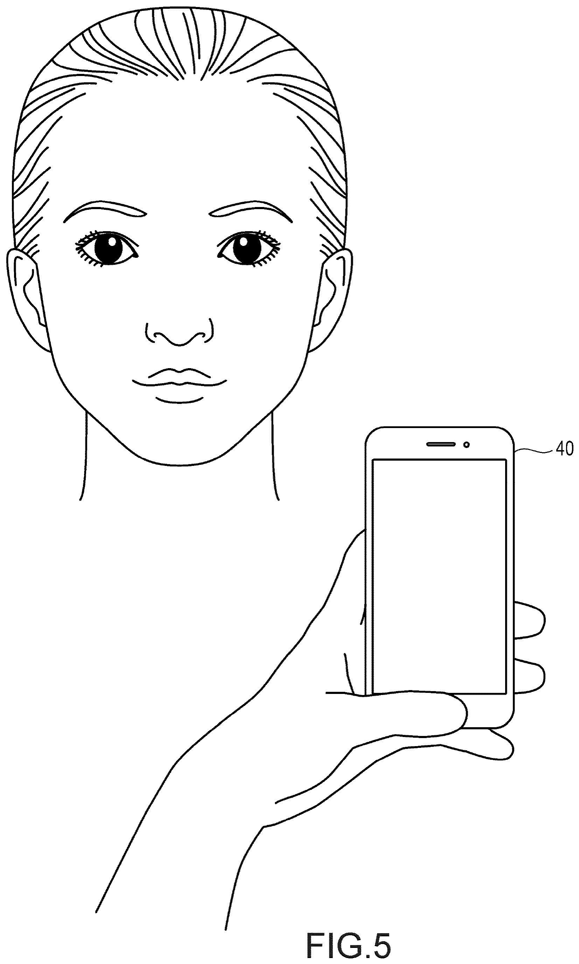

The seven skin condition indicators are acquired as skin condition indicators in Embodiment 2. As shown in Fig. 5, these skin condition indicators can be acquired on the basis of a skin image (bare skin image), which is captured by imaging the skin (bare skin) of the user with an imaging device 40, and the skin condition indicator mathematical model. The imaging device 40 used in the present embodiment can be any type of imaging device. The imaging device 40 may be an independent digital camera or video camera. Otherwise, the imaging device 40 may be an imaging device provided in a portable phone, a tablet terminal, or a personal computer, which is generally called a mobile terminal. Since the skin image (bare skin image) of the user is for discriminating skin appearance states or attributes, the imaging device 40 is favorably a camera capable of capturing visible light and producing a color image.

In this manner, the skin condition indicators are evaluation items (seven items described above) acquired by using the skin image obtained by imaging the skin of the user (bare skin image) and the skin condition indicator mathematical model indicating the relationship between the skin image and the skin condition indicators.

Here, a method of generating the skin condition indicator mathematical model will be described.

Training data used for generating the skin condition indicator mathematical model is data combining facial images of bare skins for a plurality of people, facial images with the predetermined cosmetic applied, and ground truth data indicating skin conditions of the human face shown in each of the facial images.

It is only sufficient that the face of the target subject is shown in the facial image by such a degree that skin conditions of the items to be estimated can be analyzed. It is only sufficient that the entire or a part of the face of the target subject is shown in the facial image. Sites other than the face of the subject, such as hair and neck, or the background may be shown in the facial image to be acquired. Moreover, those may be color images or may be grayscale images as long as the skin conditions of the human face shown in the facial image can be estimated. Moreover, each of them may be normalized into a predetermined rectangle shape, may have an image size different from a predetermined image size of the predetermined rectangle shape, or may include a facial image in a shape different from the predetermined rectangle shape.

The predetermined cosmetic can be any type of cosmetic. The predetermined cosmetic may or does not need to include the UV absorbing agents or the UV scattering agents. In the present embodiment, a foundation was used.

The ground truth data is information indicating skin conditions of the human face performed by an expert evaluator evaluating an associated facial image or an object itself shown in the facial image by eyes or with the measurement instrument or the like. In the present embodiment, information regarding the above-mentioned skin condition indicators (seven items) that five expert evaluators have evaluated the facial images by eyes.

### - Overall evaluation (overall skin condition level)

Evaluation of the overall skin condition level on a 7-point scale
- Skin tone (skin brightness) Evaluation of the skin brightness on a 7-point scale
- Makeup intensity (whether or not the skin looks well made up) Evaluation as to whether or not the skin looks well made up- Skin age (indicator indicating a skin aging degree) Evaluation of the skin aging degree in on a 5-point scale
- Masculinity/femininity (whether the skin texture is closer to that of a male or female) Evaluation as to whether the skin texture is closer to that of a male or female
- Makeup smudge level (indicator indicating the extent to which makeup smudges) Evaluation as to whether or not the extent to which makeup smudges is higher
- Powderiness (indicator indicating a silky smooth feel of the skin) Evaluation as to whether or not the silky smooth feel of the skin is obtained

It should be noted that in the present embodiment, evaluations were made with respect to those seven items for each of both images of the facial image of the bare skin and the facial image with the predetermined cosmetic applied.

Next, with respect to the acquired facial image, position coordinates (predetermined position coordinates) of a plurality of predetermined parts in the facial area of a human face shown in a target facial image is determined for each facial image. The predetermined position coordinates at least include position coordinates of shape feature points of the facial area.

Next, the positions of the respective pixels of the target facial image is converted so that that facial area becomes the predetermined rectangle shape based on the plurality of predetermined position coordinates determined and that the positions of the respective pixels of the original facial image are positions associated with input rules of the skin condition indicator mathematical model.

Then, the skin condition indicator mathematical model is generated by using the training data including the converted facial image. Although the learning algorithm is not limited, in the present embodiment, it was performed by deep learning.

In this manner, the "skin condition indicator mathematical model" used in the present embodiment is generated by using both images of the facial image of the bare skin and the facial image with the predetermined cosmetic applied and evaluation results of performing evaluations with respect to the above-mentioned seven items for each of both images.

It should be noted that the above-mentioned skin condition indicators (seven items) are indicators suitable for estimating a formation state of a coating film formed on the skin by the cosmetic when assuming that the cosmetic containing the UV absorbing agents or the UV scattering agents is applied in the present embodiment, so the skin condition indicator mathematical model for calculating the indicators is used. However, to estimate a formation state of a coating film formed on the skin by the cosmetic when assuming that a cosmetic other than the cosmetic containing the UV absorbing agents or the UV scattering agents is applied, it is only sufficient to use a skin condition indicator mathematical model capable of calculating skin condition indicators suitable for estimating the formation state of the coating film formed on the skin by that cosmetic.

Moreover, the "skin condition indicator mathematical model" may generate either one or both of the facial image of the bare skin and the skin condition indicator mathematical model (bare skin) generated by using the evaluation results of performing evaluations with respect to the above-mentioned seven items for the facial image of the bare skin and the facial image with the predetermined cosmetic applied and the skin condition indicator mathematical model (application) generated by using the evaluation results of performing evaluations with respect to the above-mentioned seven items for the facial image with the predetermined cosmetic applied and acquire the skin condition indicators (seven items) by using either one or both of them. It should be noted that in Embodiment 2, the skin image (bare skin image) of the user is used when acquiring the skin condition indicators of the user, so it is favorable to at least include the facial image of the bare skin and the skin condition indicator mathematical model (bare skin) including the evaluation results of performing evaluations with respect to the seven items for the facial image of the bare skin.

Next, the coating film estimation mathematical model 2 will be described.

Training data used for generating the coating film estimation mathematical model 2 and conditions thereof are as follows.

| | |
|---|---|
| - Predetermined composition 20: | cosmetic C (SPF50+, formulation type: O/W) |
| - Application area | : 4 cm × 4 cm |
| - Application amount | : 16 mg for 4 cm × 4 cm |
| - Target subject | : foreheads or cheeks of 20 females |
| - Used camera | : UV camera 30 |
| - Application method | : a trained examiner applies them uniformly. |
| - Light source | : MAX-303, which is manufactured by Asahi Spectra Co., Ltd. |
| - Bandpass filter (320 nm ±5 nm) | |
| - Polarization plate | |

As shown in Fig. 2-1, the predetermined composition 20 is applied to the skin of the subject, the skin of the subject is imaged with the UV camera 30 under UV irradiation, and the thus captured image data (UV skin image) is used. The coating film estimation mathematical model 2 is generated from the captured image data by the following procedure. It should be noted that the acquisition method for the UV reflectance of the predetermined composition 20 (cosmetic C) is similar to that of Embodiment 1.

1) Calculate a UV reflectance of the coating film of the cosmetic formed on the skin

A UV reflectance (referred to as a "UV reflectance C") of a coating film formed by the cosmetic C on the skin when the predetermined composition 20 (cosmetic C) is applied to the skin (skin 15) of the subject is calculated. The calculation method is shown below.

Fig. 3 (a) shows a case where the skin (bare skin) of the subject is irradiated with UV rays (hereinafter, "UV light") with a wavelength of 320 nm included in the UVB region. The "incident light" in the figure is the irradiated UV light, the "positive reflection light" is UV light reflected on the surface of the skin, and the "internal scattered light" is UV light, which has not been reflected on the surface of the skin, entered the skin, spread inside the skin, and went out of the skin the UV light. The intensity of the internal scattered light, from which the positive reflection light is removed, is acquired. This is handled as internal scattered light intensity of the bare skin.

Fig. 3 (b) shows a case where the predetermined composition 20 is applied to the skin of the subject and is irradiated with UV light. The "incident light" in the figure is the irradiated UV light, the "positive reflection light" is UV light reflected on the surface of the predetermined composition 20 and UV light reflected on the surface of the skin. The "internal scattered light" is UV light, which has been not reflected on the surface of the predetermined composition 20 and the surface of the skin enters the skin, spread inside the skin, and went out of the skin the UV light. The intensity of internal scattered light, from which the positive reflection light in a case where the cosmetic C is applied as the predetermined composition 20 is removed, is acquired. This is handled as internal scattered light intensity C of the coated skin.

Then, by dividing the internal scattered light intensity C of the coated skin when the predetermined composition 20 is applied by the internal scattered light intensity of the bare skin, a UV reflectance of the composition present in the site to which the predetermined composition 20 is applied is calculated (the internal scattered light intensity C of the coated skin to which cosmetic C is applied/the internal scattered light intensity of the bare skin = the UV reflectance of the coating film (UV reflectance C) formed by the cosmetic C present in the site to which cosmetic C is applied.

In this manner, the positive reflection light is removed and the UV reflectance is calculated with the internal scattered light intensity. Accordingly, it is possible to estimate an application state of the coating film (formation state of the coating film) formed on the skin with high accuracy.

Here, the internal scattered intensity can be measured by mounting polarization plates on the light source and the UV camera, thereby irradiating the skin to which the cosmetic C is applied is applied with UV light, removing the positive reflection light, and capturing an image of the reflected internal scattered light with the UV camera 30.

It should be noted that in the present embodiment, the UV reflectance with a wavelength of 320 nm is calculated in view of the fact that the wavelength range of the UVB region is 320 to 280 nm, though the wavelength range is not limited thereto. For example, in a case of estimating a formation state of the coating film formed on the skin by the cosmetic and estimating and evaluating the UV protection effect of the UVA region from the estimation result, the reflectance of UV rays may be calculated by using UV rays with any one of wavelengths of 380 to 320 nm, e.g., a wavelength of 350 nm.

### 2) Sort into two clusters in accordance with the UV reflectance

The calculated UV reflectances of the coating films formed on the skin by the respective cosmetics are arranged in order of the UV reflectance and the respective subjects are sorted into two clusters, that with a higher UV reflectance (50%) and that with a lower UV reflectance (50%). The cluster whose UV reflectance is higher will be referred to as a "cluster whose level of the formation state of the coating film of the cosmetic is lower" and the cluster whose UV reflectance is lower will be referred to as a "cluster whose level of the formation state of the coating film of the cosmetic is higher." It should be noted that the reason why the UV reflectances of the coating films formed on the skins by the respective cosmetics, which are calculated by the subjects, differ (can be ordered) is because the coating film of each cosmetic differs depending on the conditions of the skin to which the cosmetic is applied. It should be noted that the point that the skin conditions of the skin to which the cosmetic is applied affect the formation state of the coating film of the cosmetic is as described above in Embodiment 1.

### 3) Measure skin condition indicators of the subject

Skin condition indicators (seven items) are acquired by using the skin condition indicator mathematical model and a captured skin image (bare skin image) of the subject.

### 4) Generate training data for use in machine learning

Training data for generating the coating film estimation mathematical model 2 is generated by using the result of sorting into the two clusters in 2) and the acquired result in 3).

### 5) Generate a coating film estimation mathematical model 2

For each evaluation value, standardization processing is performed, optimal one is selected from a plurality of classification models (e.g., naive bayes (NB), logistic regression (LR), linear discriminant analysis (LDA), random forest (RF), gradient boosting (GB), support vector machine (SVM), Gaussian process (GP)) and set as a prediction model, and whether it is sorted into a cluster whose level of the formation state of the coating film of the cosmetic is lower or a cluster whose level is higher is predicted by using this prediction model. A matching degree of the predicted cluster and the actual cluster is calculated as an accuracy rate. The generalization performance of the classification model is evaluated by leave-one-out cross-validation (LOOCV) and a calculation model having the highest accuracy rate is set as an optimal model: coating film estimation mathematical model **2.** It should be noted that in the present embodiment, a classification model RF was used for the coating film estimation mathematical model 2 of the cosmetic C.

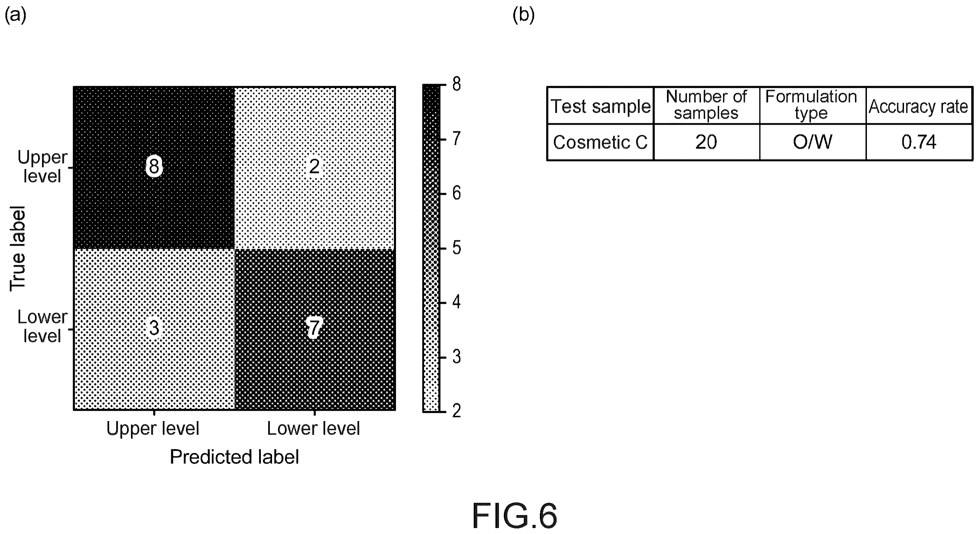

Fig. 6 (a) shows results of predicting whether it is sorted into the cluster whose level of the formation state of the coating film formed on the skin by the cosmetic is lower or the cluster whose level is higher by using the coating film estimation mathematical model 2 and actual results. Fig. 6 (b) shows results of calculating a matching degree of the cluster predicted and the actual cluster as an accuracy rate by using the coating film estimation mathematical model 2.

It can be seen from Fig. 6 (a) and (b) that a high accuracy rate is obtained in a case of predicting the formation state of the coating film formed on the skin by the cosmetic by using the coating film estimation mathematical model 2.

Therefore, by using the skin condition indicators of the user estimated by using the skin condition indicator mathematical model and the coating film estimation mathematical model 2, the formation state of the coating film formed on the skin by the composition can be estimated without applying the predetermined composition to the skin. Therefore, it is possible to estimate whether the level of the formation state of the coating film formed on the skin by the composition is high or low when the predetermined composition is applied to the skin of the user even without applying the predetermined composition (cosmetic to which the UV absorbing agents and/or the UV scattering agents have been added) to the skin. Accordingly, it is also possible to evaluate to which extent an effect based on a function of the predetermined cosmetic (e.g., a UV protection function in a case of the cosmetic to which the UV absorbing agents and/or the UV scattering agents have been added) can be expected.

As described above, Embodiment 2 includes generating a mathematical model (coating film estimation mathematical model 2) indicating a relationship between the skin condition indicators and UV reflectances of the coating film of the cosmetic by using the skin condition indicators (e.g., the seven items calculated by using the skin image of the subject and the skin condition indicator mathematical model) of each of the plurality of subjects (e.g., 20 females) and the UV reflectance of the coating film formed on the skin by the cosmetic, which is calculated from UV skin images (e.g., the images shown in Fig. 2-2 captured by the imaging device shown in Fig. 2-1) of each of the plurality of subjects. It is possible to estimate a formation state of the coating film formed on the skin by the cosmetic when assuming that the cosmetic is applied to the skin of the user by using the generated coating film estimation mathematical model 2 and the skin condition indicators (e.g., the above-mentioned seven items) of the user, which are acquired from the skin image (bare skin image) of the user and the skin condition mathematical model, and it is possible to estimate and evaluate the function exerted by the cosmetic.

Moreover, as in Embodiment 1, the formation state of the coating film formed on the skin by the cosmetic is estimated by using the reflectance of the UV light with a wavelength of 320 nm in the wavelength range of UVB, so it is possible to directly estimate and evaluate the function of the cosmetic (the UV protection function in the UVB region for a cosmetic with a UV protection function in the UVB region) on the basis of the formation state of the coating film formed on the skin by the cosmetic. Specifically, for example, in a case of the cosmetic C (SPF50+), a UV reflectance of each of the plurality of subjects (20 subjects) is calculated and a standard value of the calculated UV reflectances is determined. The "standard value" in the present embodiment is a UV reflectance located at the center when the UV reflectances are arranged in order of the UV reflectance. It has been described in "2) Sort into two clusters in accordance with the UV reflectance." Then, the following estimations can be made. Specifically, in a case where the formation state (UV reflectance) of the coating film formed on the skin by the cosmetic of the subject, which is estimated by the above-mentioned method, is equal to the standard value, the effect claimed for the cosmetic C (equivalent to SPF50+) can be expected. In a case where it is greater than the standard value, the effect claimed for the cosmetic C (equivalent to SPF50+) can be expected to be sufficient. In a case where it is less than the standard value, the effect claimed for the cosmetic C (equivalent to SPF50+) cannot be expected to be sufficient. Thus, the user can judge whether or not that cosmetic is suitable to the user's skin on the basis of the estimated formation state of the coating film formed on the skin by the cosmetic.

Moreover, as in Embodiment 1, by generating the above-mentioned coating film estimation mathematical model 2 where the first time zone is, for example, 1 minute later after application and the second time zone is, for example, 8 hours later after application, it is possible to estimate the first formation state and the second formation state. It should be noted that it is only sufficient that in the generation method, as in Embodiment 1, the process of "1) Calculate a UV reflectance of the coating film of each cosmetic formed on the skin" described in Embodiment 2 above is performed, UV reflectances of each cosmetic are calculated when 1 minute has elapsed since the predetermined cosmetic was applied to the skins of the subjects and when 8 hours have elapsed since the predetermined cosmetic was applied to the skins of the subjects, respectively, the above-mentioned processes of "2) Sort into two clusters in accordance with the UV reflectance" to "5) Generate a coating film estimation mathematical model 2" are performed, and the coating film estimation mathematical model 2 to estimate the formation state (first formation state) of the coating film formed on the skin by the cosmetic 1 minute later after application and the coating film estimation mathematical model 2 to estimate the formation state (first formation state) of the coating film formed on the skin by the cosmetic 8 hours later after application are configured.

In this manner, the coating film estimation mathematical model 2 to estimate the formation state (first formation state) of the coating film formed on the skin by the cosmetic 1 minute later after application and the coating film estimation mathematical model 2 to estimate the formation state (second formation state) of the coating film formed on the skin by the cosmetic 8 hours later after application are generated, and the two generated coating film estimation mathematical models 2 and the skin condition indicators of the user that are acquired by the predetermined measurement instrument are used. Accordingly, it is possible to estimate a formation state of the coating film formed on the skin by the cosmetic after the predetermined time since application without applying the predetermined cosmetic to the skin of the user and even when a predetermined time (in the present embodiment, the first time zone: 1 minute or the second time zone: 8 hours) has not elapsed since application.

In this manner, it is possible to estimate the formation state (first formation state) of the coating film formed on the skin by the composition in the first time zone (e.g., 1 minute after application) after application in which the predetermined composition (e.g., cosmetic C) is applied and the formation state (second formation state) of the coating film formed on the skin by the composition in the second time zone later than the first time zone (e.g., 8 hours after application). It should be noted that it is not necessarily necessary to estimate both the first formation state and the second formation state, and only either one of them may be estimated depending on a purpose. Moreover, it is also possible to increase the number of time zones, for example, a third time zone, a fourth time zone, and so on.

Moreover, as in Embodiment 1, it is possible to estimate each of an effect that can be expected in the first time zone and an effect that can be expected in the second time zone with respect to the effect claimed for the applied composition by using the first formation state and the second formation state.

As described above, the predetermined cosmetic is the cosmetic (e.g., cosmetic C) to which the UV absorbing agents and/or the UV scattering agents have been added, and it is possible to estimate the first formation state and the second formation state and estimate and evaluate the function exerted by the cosmetic on the basis of the estimated first formation state and second formation state (evaluate to which extent the effect can be expected with respect to the function of the cosmetic).

Therefore, for example, if the user wishes to estimate and evaluate the UV protection effect in a case where the user applies the cosmetic before go-out and enjoys activity in a circumstance where the user is easily exposed to UV rays 8 hours later, it is only sufficient to estimate the formation state of the coating film formed on the skin by the cosmetic in the second time zone. Moreover, for example, if the user wishes to estimate the UV protection effect in a case where the user applies the cosmetic before go-out and enjoys activity continuously for 8 hours in a circumstance where the user is easily exposed to UV rays, it is possible to estimate both formation states of the coating films formed on the skin by the cosmetic in the first time zone and the second time zone and estimate and evaluate it by using both. In this manner, it is possible to use the coating film estimation mathematical model 2 depending on each purpose.

### <Embodiment 3>

Embodiment 3 is a method of estimating a formation state of a coating film formed on a skin by a composition formed when a predetermined composition is applied on the basis of skin condition indicators generated from an image obtained by imaging a skin (bare skin) of a user by using a mathematical model and a mathematical model indicating a relationship between the skin condition indicators and the formation state of the coating film of the composition when assuming that the predetermined composition is applied to the skin.

In the present embodiment, the mathematical model for use for generating the skin condition indicators will be referred to as a "skin condition indicator mathematical model" and is identical to the "skin condition indicator mathematical model" described in Embodiment 2 above. The mathematical model indicating the relationship between the skin condition indicators acquired and the formation state of the coating film of the composition when assuming that the predetermined composition is applied to the skin will be referred to as a "coating film estimation mathematical model 3." In Embodiment 3, the formation state of the coating film formed on the skin by the composition is estimated by using the "skin condition indicator mathematical model" and the "coating film estimation mathematical model 3" without applying the composition to the skin of the user.

The skin condition indicator mathematical model and the coating film estimation model 3 will be described.

The "skin condition indicator mathematical model" is a learned discrimination model learned on the basis of a plurality of pieces of training data and is a discrimination model learned to be capable of acquiring at least seven skin condition indicators as follows and is similar to the "skin condition indicator mathematical model" described in Embodiment 2 above.
- Overall evaluation (overall skin condition level)
- Skin tone (skin brightness)
- Makeup intensity (whether or not the skin looks well made up)
- Skin age (indicator indicating a skin aging degree)
- Masculinity/femininity (whether the skin texture is closer to that of a male or female)
- Makeup smudge level (indicator indicating the extent to which makeup smudges)
- Powderiness (indicator indicating a silky smooth feel of the skin)

It should be noted that since the "skin condition indicator mathematical model" is similar to the "skin condition indicator mathematical model" described in Embodiment 2 above, it is generated by using each of both images of the facial image of the bare skin and the facial image with the predetermined cosmetic applied and evaluation results of performing evaluations with respect to the above-mentioned seven items for each of both images. A description of the generation method for the "skin condition indicator mathematical model" will be omitted.

Next, the coating film estimation mathematical model 3 will be described.

The training data used for generating the coating film estimation mathematical model 3 and conditions thereof are as follows.

| | |
|---|---|
| - Predetermined composition 20: | cosmetic D (SPF50+, formlation type: O/W) |
| | cosmetic E (SPF50+, formulation type: W/O) |
| - Application area | : full face |
| - Application amount | : 300 mg for full face |
| - Target subject | : full faces of 20 females for both cosmetic D and cosmetic E |
| - Used camera | : visible light camera |
| - Application method | : subjects apply them on the full face uniformly |

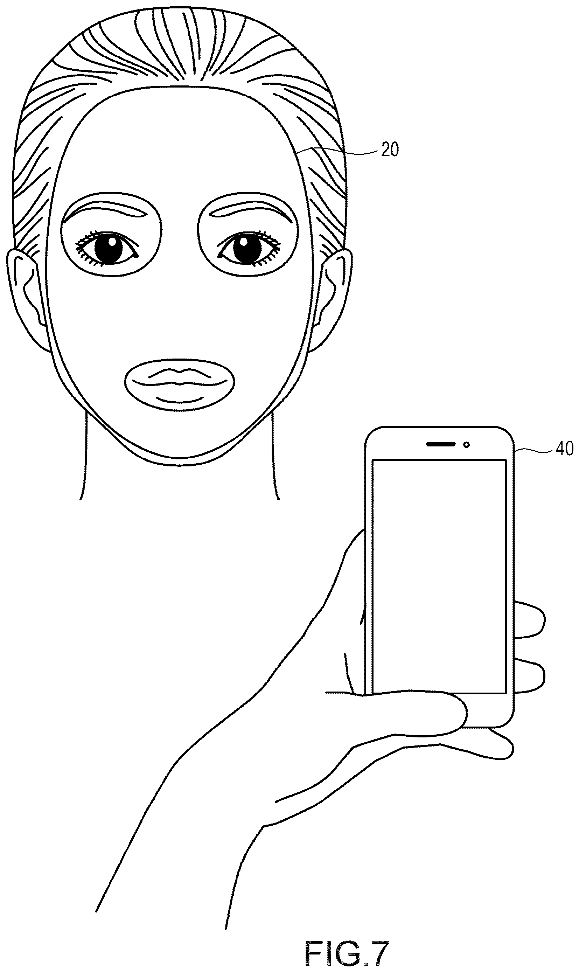

As shown in Fig. 7, the predetermined composition 20 (cosmetic D or cosmetic E) is applied to the skin of the subject and image data (coated skin image) captured by the imaging device 40 (smartphone 40) is used. It should be noted that the imaging device 40 (smartphone 40) can be any type of imaging device. The imaging device 40 may be a digital camera or a video camera. Otherwise, the imaging device 40 may be an imaging device provided in a portable phone, a tablet terminal, and a personal computer, which is generally called a mobile terminal. The imaging device 40 is favorably a camera of capable of capturing a color image, which captures visible light. In the present embodiment, the imaging is performed by a camera mounted on a smartphone. The coating film estimation mathematical model 3 is generated from the captured image data (coated skin image) by the following procedure.

### 1) Acquire an evaluation value of a coated skin overall evaluation

As described above, the coated skin images of the full faces of the plurality of subjects (in the present embodiment, 20 females) to which each of the cosmetic D and the cosmetic E is applied are acquired. An evaluation value of a coated skin overall evaluation is acquired by using each of the acquired coated skin images of the full faces and the "skin condition indicator mathematical model."

The "evaluation value of the coated skin overall evaluation" is an evaluation value of the "overall evaluation" of the skin condition indicators (seven items described above (overall evaluation, skin tone, makeup intensity, skin age, masculinity/femininity, degree of makeup smudging, and powderiness)), which is calculated by using the acquired coated skin image of the full face and the "skin condition indicator mathematical model." In the present embodiment, in order to distinguish it from the "overall evaluation" as one item of "skin condition indicators" calculated by using the skin image (bare skin image) of the user and the "skin condition indicator mathematical model," the "overall evaluation" calculated by using the coated skin image of the full face to which the cosmetic D (or cosmetic E) is applied and the "skin condition indicator mathematical model" will be referred to as a "coated skin overall evaluation." Moreover, the evaluation value of the coated skin overall evaluation will be referred to as a "coated skin overall evaluation value."

### 2) Sort into two clusters in accordance with the coated skin overall evaluation value

The coated skin overall evaluation values of each cosmetic, which have been calculated from the respective subjects, are arranged in order of the coated skin overall evaluation value and are sorted into two clusters, that with a higher coated skin overall evaluation value (50%) and that with a lower coated skin overall evaluation value (50%). The cluster whose coated skin overall evaluation value is higher will be referred to as a "cluster whose level of the formation state of the coating film of the cosmetic is higher" and the cluster whose coated skin overall evaluation value is lower will be referred to as a "cluster whose level of the formation state of the coating film of the cosmetic is lower."

### 3) Measure skin condition indicators of the subject

Skin condition indicators (seven items) are acquired by using the skin condition indicator mathematical model described in Embodiment 2 above with respect to the subject and a skin (bare skin) image of the subject captured by the visible light camera.

### 4) Generate training data for use in machine learning

Training data is generated by using the result of sorting into the two clusters in 2) and the acquired result in 3).

### 5) Generate a coating film estimation mathematical model 3

For each evaluation value, standardization processing is performed, optimal one is selected from a plurality of classification models (e.g., naive bayes (NB), logistic regression (LR), linear discriminant analysis (LDA), random forest (RF), gradient boosting (GB), support vector machine (SVM), Gaussian process (GP)) and set as a prediction model, and whether it is sorted into a cluster whose level of the formation state of the coating film of the cosmetic is lower or a cluster whose level is higher is predicted by using this prediction model. A matching degree of the predicted cluster and the actual cluster is calculated as an accuracy rate. The generalization performance of the classification model is evaluated by leave-one-out cross-validation (LOOCV) and a calculation model having the highest accuracy rate is set as an optimal model: coating film estimation mathematical model 3. It should be noted that in the present embodiment, a classification model NB was used for the coating film estimation mathematical model 3 of the cosmetic D and a classification model LDA was used for the coating film estimation mathematical model 3 of the cosmetic E.

In this manner, the coating film estimation mathematical model 3 is generated by learning with the "skin condition indicators (seven items)" generated by using the skin condition indicator mathematical model and the skin images (bare skin image) of the plurality of subjects and the "coated skin overall evaluation value" generated by using a skin condition indicator inference model and the coated skin images of the full faces where the cosmetic D (or cosmetic E) is applied to the skins of the plurality of subjects. The skin condition indicators (seven items) include items that vary depending on the physical property values of the skin (e.g., the stratum corneum water content, the moisture transpiration value, etc.) that affect the formation of the coating film of the cosmetic on the skin. Therefore, as a result of further research, it was found that using all skin condition indicators (seven items) to be calculated can generate a coating film estimation mathematical model 3 with a high accuracy rate. Moreover, although using the coated skin images of the full faces where the cosmetic D (or cosmetic E) is applied to the skin of the subject and the skin condition indicator inference model can calculate items similar to the skin condition indicators (seven items), only the "coated skin overall evaluation value" that is the evaluation value of the coated skin overall evaluation corresponding to the overall evaluation was used in the present embodiment. It is because the formation state of the coating film formed on the skin is estimated by applying the cosmetic to the skin in this method, and as a result of further research, it was found that an evaluation result of a skin appearance state to which the cosmetic has been applied, a finished state, is the most important in estimating the formation state of the coating film formed on the skin, and as a result, it was found that that using the "coated skin overall evaluation value" that is the evaluation value of the overall evaluation that is the overall skin condition level can generate a coating film estimation mathematical model with a high accuracy rate.

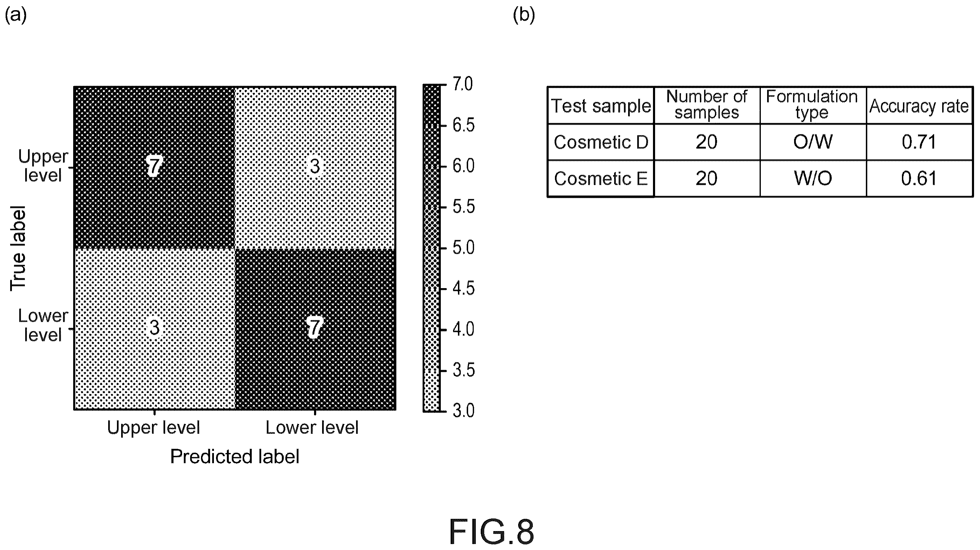

Fig. 8 (a) shows results of predicting whether it is sorted into a cluster whose level of the formation state of the coating film of the cosmetic is lower or a cluster whose level is higher by using the coating film estimation mathematical model 3 and actual results. Fig. 8 (b) shows results of calculating the matching degree of the cluster predicted by using the coating film estimation mathematical model 3 and an actual cluster as an accuracy rate.

It can be seen from Fig. 8 (a) and (b) that a high accuracy rate is obtained in a case of predicting the formation state of the coating film of the cosmetic by using the coating film estimation mathematical model 3.

Therefore, it is possible to estimate a formation state of the coating film of the composition by the predetermined composition by using the skin condition indicators of the user (seven items) acquired by using the skin condition indicator mathematical model and the coating film estimation mathematical model 3. Therefore, it is possible to estimate whether the level of the formation state of the coating film is high or low when assuming that the predetermined composition is applied the skin of the user even without applying the predetermined composition (cosmetic to which the UV absorbing agents and/or the UV scattering agents have been added) to the skin. Accordingly, it is also possible to estimate to which extent an effect based on a function of the predetermined composition (e.g., a UV protection function in a case of the cosmetic to which the UV absorbing agents and/or the UV scattering agents have been added) can be expected.

As described above, Embodiment 3 includes acquiring each of the skin condition indicators (e.g., the seven items calculated by using the skin image (bare skin image) of the subject and the skin condition indicator mathematical model) of the skin of each of the plurality of subjects (e.g., 20 females). The coated skin overall evaluation value is calculated by using the skin images obtained by imaging the skins (coated skins) of the plurality of subjects to which the cosmetic (e.g., cosmetic D or cosmetic E) is applied (e.g., skin images (coated skin images) captured by the imaging device shown in Fig. 7) and the skin condition indicator mathematical model. The mathematical model (coating film estimation mathematical model 3) indicating the relationship between the skin condition indicators and the formation state of the coating film of the cosmetic is generated from the acquired skin condition indicators (seven items) and the coated skin overall evaluation value. It is possible to estimate a formation state of the coating film formed on the skin by the cosmetic when assuming that the cosmetic (e.g., cosmetic D or cosmetic E) is applied to the skin of the user by using the skin condition indicators (e.g., the above-mentioned seven items) of the user, which are acquired from the skin image (bare skin image) of the user and the skin condition mathematical model, and the coating film estimation mathematical model 3, and it is possible to estimate and evaluate the function exerted by the cosmetic.

Moreover, as in Embodiment 1 or 2, by generating the above-mentioned coating film estimation mathematical model 3 where the first time zone is, for example, 1 minute later after application and the second time zone is, for example, 8 hours later after application, it is possible to estimate the first formation state and the second formation state. It should be noted that it is only sufficient that in the generation method, as in Embodiment 1 or 2, the process of "1) Acquire an evaluation value of each coated skin overall evaluation" described in Embodiment 3 above is performed, evaluation values of the respective coated skin overall evaluations are calculated when 1 minute has elapsed since the predetermined cosmetic was applied to the skins of the subjects and when 8 hours have elapsed since the predetermined cosmetic was applied to the skins of the skins of the subjects, respectively, the processes of "2) Sort into two clusters in accordance with the evaluation value of the coated skin overall evaluation value" to "5) Generate a coating film estimation mathematical model 3," which have been described Embodiment 3, are performed, and the coating film estimation mathematical model 3 to estimate the formation state (first formation state) of the coating film of the cosmetic 1 minute later after application and the coating film estimation mathematical model 3 to estimate the formation state (second formation state) of the coating film of the cosmetic 8 hours later after application are configured.

In this manner, the coating film estimation mathematical model 3 to estimate the formation state (first formation state) of the coating film formed on the skin by the cosmetic 1 minute later after application and the coating film estimation mathematical model 3 to estimate the formation state (second formation state) of the coating film formed on the skin by the cosmetic 8 hours later after application are generated, and the two generated coating film estimation mathematical models 3 and the skin condition indicators of the user, which have been acquired by using the skin condition indicator mathematical model are used. Accordingly, it is possible to estimate a formation state of the coating film formed on the skin by the cosmetic after the predetermined time since application without applying the predetermined cosmetic to the skin of the user and even when a predetermined time (in the present embodiment, the first time zone: 1 minute or the second time zone: 8 hours) has not elapsed since application.

In this manner, it is possible to estimate the formation state (first formation state) of the coating film formed on the skin by the composition in the first time zone (e.g., 1 minute after application) after application in which the predetermined composition (e.g., cosmetic D or cosmetic E) is applied and the formation state (second formation state) of the coating film formed on the skin by the composition in the second time zone later than the first time zone (e.g., 8 hours after application). It should be noted that it is not necessarily necessary to estimate both the first formation state and the second formation state, and only either one of them may be estimated depending on a purpose. Moreover, it is also possible to increase the number of time zones, for example, a third time zone, a fourth time zone, and so on.

Moreover, as in Embodiment 1 or 2, it is possible to estimate each of an effect that can be expected in the first time zone and an effect that can be expected in the second time zone with respect to the effect claimed for the applied cosmetic by using the first formation state and the second formation state.

As described above, the predetermined cosmetic is the cosmetic (e.g., cosmetic D or E) to which the UV absorbing agents and/or the UV scattering agents have been added, and it is possible to estimate the first formation state and the second formation state and estimate and evaluate the function exerted by the cosmetic on the basis of the estimated first formation state and second formation state (evaluate to which extent the effect can be expected with respect to the function of the cosmetic).

Next, a method of evaluating suitability of a cosmetic with a predetermined function to the skin of the user by using the formation state of the coating film formed on the skin by the cosmetic, which is estimated by each of the methods in Embodiments 1 to 3, will be described.

As described above, in all embodiments, the formation state of the coating film formed on the skin by the cosmetic when it is actually applied is estimated without actually applying the cosmetic to which the UV absorbing agents and/or the UV scattering agents have been added to the skin of the user. The predetermined cosmetic is the cosmetic to which the UV absorbing agents and/or the UV scattering agents have been added and the formation state of the coating film formed on the skin by the cosmetic is estimated on the basis of the UV reflectance with a wavelength of 320 nm included in the UVB region in Embodiments 1 and 2. Therefore, it is possible to estimate whether or not the effect of protection from UV rays in the UVB wavelength range that the predetermined cosmetic has can be expected.

Moreover, as described above, it is also possible to estimate the first formation state that is the formation state of the coating film formed on the skin by the cosmetic in the first time zone (e.g., 1 minute later after application) and the second formation state that is the formation state of the coating film formed on the skin by the cosmetic in the second time zone (e.g., 8 hours later after application). Therefore, it is possible to respectively estimate an effect that can be expected in the first time zone and an effect that can be expected in the second time zone with respect to the effect claimed for the applied cosmetic by using the first formation state and the second formation state.

Then, a method of proposing cosmetics suitable to the user by using these estimation results will be described.

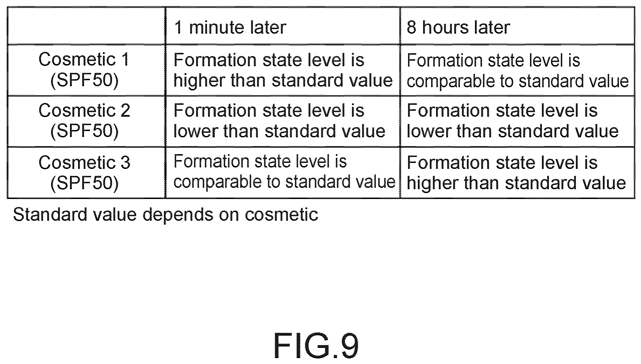

Fig. 9 shows an example showing whether each of the first formation state in the first time zone (1 minute later) and the second formation state in the second time zone (8 hours later), which are estimated in a case where three types of cosmetics, Cosmetics 1 to 3, are applied, is higher/lower than a standard value.

Here, Cosmetics 1 to 3 all have the same published UV protection value (in the present embodiment, all SPF50). In this method, it is possible to estimate whether or not the effect claimed for the cosmetic can be expected by estimating a formation state of a coating film formed on the skin by the cosmetic when assuming that the cosmetic is applied. Therefore, it is effective to compare cosmetics with the same published UV protection value. It should be noted that since the standard value depends on a cosmetic, it cannot be said that a similar UV protection value can be expected for example even if Cosmetic 1 and Cosmetic 2 are both comparable to the standard value, though it is effective as information when proposing cosmetics suitable to the user.

According to Fig. 9, for example the first formation state and the second formation state for Cosmetic 2 are both lower than the standard value. Therefore, it can be estimated that it is difficult for the user to uniformly apply it. Thus, it can be estimated that it is difficult to expect the effect claimed for Cosmetic 2. Moreover, for Cosmetic 1, the first formation state is higher than the standard value and the second formation state is comparable to the standard value while for Cosmetic 3, the first formation state is comparable to the standard value and the second formation state is higher than the standard value. Therefore, it is only sufficient to propose Cosmetic 1 if the user wishes to get the UV protection effect immediately after the user applies the cosmetic, and to propose Cosmetic 3 if the user applies it before go-out and goes to a location where the user is exposed to UV rays a predetermined time later. In this manner, it is possible to use useful information when proposing cosmetics.

As described above, the cosmetic (e.g., Cosmetics 1 to 3) has the published UV protection value published in advance (e.g., SPF value). It is possible to estimate and evaluate the UV protection function as a predetermined function on the basis of the estimated formation state of the coating film formed on the skin by the cosmetic and the above-mentioned published UV protection value.

### <Estimation Apparatus>

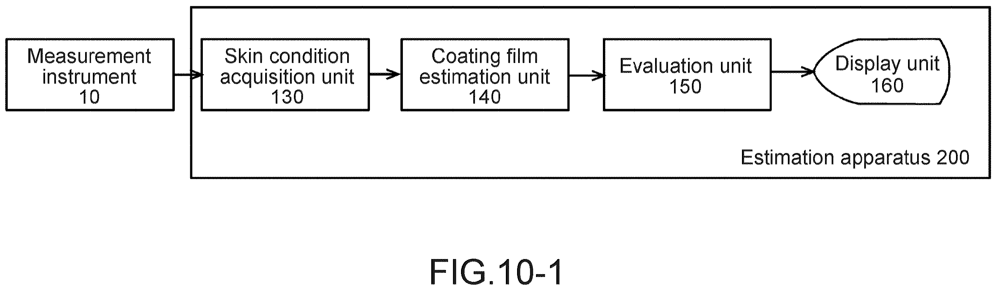

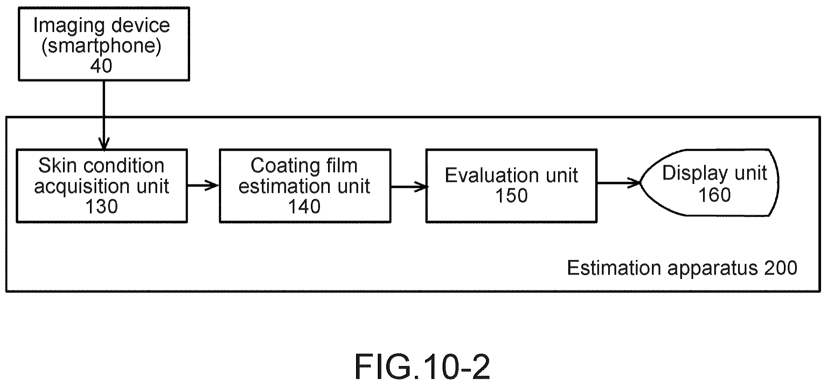

An estimation apparatus 200 will be described with reference to Figs. 10-1 and 10-2. Fig. 10-1 shows the case of Embodiment 1. Fig. 10-2 shows the case of Embodiment 2 or 3.

The estimation apparatus 200 in the present embodiment is constituted by a skin condition acquisition unit 130 and a coating film estimation unit 140. The estimation apparatus 200 is a general-purpose personal computer. The estimation apparatus 200 includes an input device including a keyboard, a pointing device, and the like, an arithmetic processing device including an MPU that is a CPU realized as an integrated circuit and the like, an input/output interface, and a storage unit, for example. The skin condition acquisition unit 130 is realized by an arithmetic processing device executing predetermined processing of the program via the input/output interface. The coating film estimation unit 140 is realized by the arithmetic processing device executing predetermined processing of the program via the input/output interface. Moreover, an evaluation unit 150 and a display unit 160 (display device) are favorably provided in the estimation apparatus 200. However, the display unit 160 may be provided outside the estimation apparatus 200 and connected via a network or the like. Moreover, in the case of Embodiment 1, as shown in Fig. 10-1, the measurement instrument 10 that measures the skin condition indicators is necessary. In the case of Embodiment 2 or 3, as shown in Fig. 10-2, the imaging device 40 that captures the skin image (bare skin image) for calculating the skin condition indicators is necessary. In a case of estimating to which extent the effect can be expected for cosmetic recommendation when assuming that the application is done without actually applying the predetermined cosmetic (cosmetic to which the UV absorbing agents and/or the UV scattering agents have been added) to the skin of the user at a store or the like, it becomes possible to sequentially propose an expected level of the UV protection effect of the predetermined cosmetic and recommended cosmetics to the user by providing the measurement instrument 10 and/or the imaging device 40 in addition to the estimation apparatus 200.

The skin condition acquisition unit 130 is a means (skin condition acquisition means) for acquiring the skin condition indicators (seven items) on the basis of the measurements obtained by the measurement instrument 10 (Embodiment 1) or by using the skin image (bare skin image) captured by the imaging device 40 and the skin condition indicator mathematical model (Embodiment 2 or 3). It should be noted that in Embodiment 2 or 3, the skin condition indicators may be calculated by using the skin image (bare skin image) obtained by imaging the user and the skin condition indicator mathematical model with an information processing terminal (not shown) different from the estimation apparatus 200 and a configuration may be made so that the calculated skin condition indicators can be acquired by the estimation apparatus 200 via a network line or medium, for example. Alternatively, the skin condition indicator mathematical model may be stored in the estimation apparatus 200, the skin image (bare skin image) captured by the imaging device 40 may be acquired, and the skin condition indicators may be calculated/acquired by the estimation apparatus 200.

The coating film estimation unit 140 is a means (coating film state estimation means) of estimating a formation state of a coating film formed on the skin by the cosmetic when assuming that a predetermined cosmetic is applied to the skin of the user by using the acquired skin condition indicators and any one of the coating film estimation mathematical models 1 to 3. The estimation method is similar to the above-mentioned one.

The evaluation unit 150 is a means of comparing and evaluating cosmetics with a published UV protection value, to which the UV absorbing agents and/or the UV scattering agents have been added, by using the formation states of the coating films formed on the skin by the cosmetics, which are estimated by the coating film estimation unit 140, and proposing a cosmetic to which the UV absorbing agents and/or the UV scattering agents have been added, which is suitable for the skin of the user. The evaluation result from the evaluation unit 150 is favorably displayed so that the user can easily get it through the display unit 160.

Displaying the evaluation result, e.g., the table shown in Fig. 9, from the evaluation unit 150 on the display unit 160 enables the user to easily get the estimation result/evaluation result.

The storage unit of the estimation apparatus 200 has stored a program for executing each of the mathematical models and the estimation method. The program causes the coating film estimation unit 140 to estimate the formation state of the coating film formed on the skin by the composition by using the skin condition indicators acquired by the skin condition acquisition unit 130 and each mathematical model, causes the evaluation unit 150 to make an evaluation, and causes the display unit 160 to display the evaluation result. It should be noted that some functions may be shared by another information processing device. For example, the respective mathematical models may be stored in another apparatus, e.g., a predetermined server (not shown), and the server may be accessed from the estimation apparatus 200 via, for example, the Internet, to cause the other apparatus to estimate the formation state of the coating film of the cosmetic by using the skin condition indicators acquired by the estimation apparatus 200.

### <Estimation System>

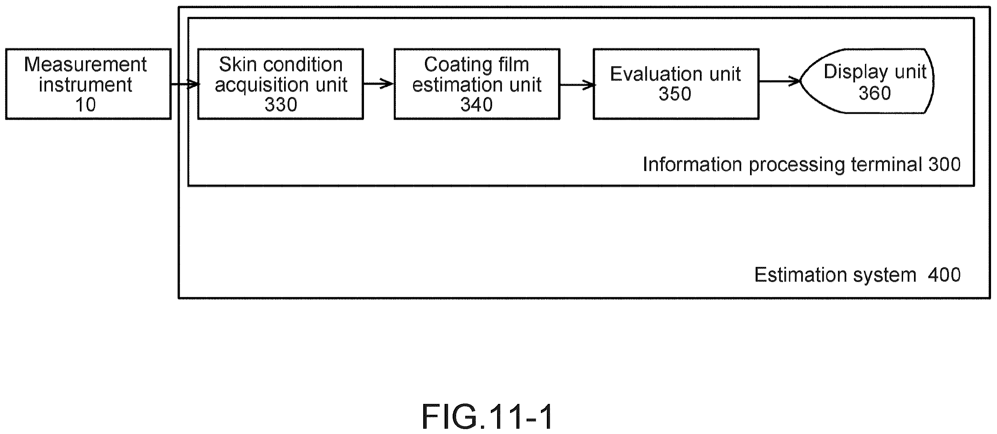

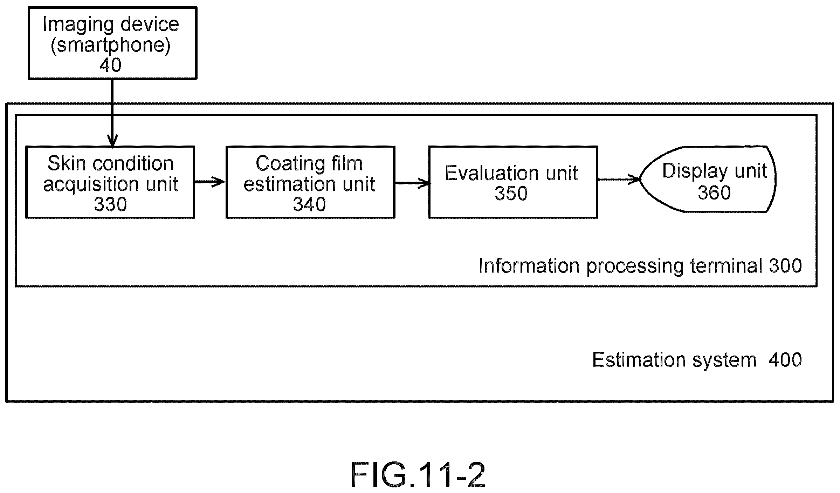

An estimation system 400 will be described with reference to Figs. 11-1 and 11-2. Fig. 11-1 shows the case of Embodiment 1. Fig. 11-2 shows the case of Embodiment 2 or 3.

The estimation system 400 in the present embodiment is constituted by a skin condition acquisition unit 330 and a coating film estimation unit 340. The estimation system 400 includes an information processing terminal 300 capable of executing various types of processing. The information processing terminal 300 includes a skin condition acquisition unit 330 and a coating film estimation unit 340. The information processing terminal 300 is a general-purpose personal computer. The information processing terminal 300 includes an input device including a keyboard, a pointing device, and the like, an arithmetic processing device including an MPU that is a CPU realized as an integrated circuit and the like, an input/output interface, and a storage unit, for example. The skin condition acquisition unit 330 is realized by the arithmetic processing device executing predetermined processing of the program via the input/output interface. The coating film estimation unit 340 is realized by the arithmetic processing device executing predetermined processing of the program via the input/output interface. Moreover, an evaluation unit 350 and a display unit 360 (display device) are favorably provided in the information processing terminal 300. However, the display unit 360 may be provided outside the information processing terminal 300 and connected via a network or the like. Moreover, in the case of Embodiment 1, as shown in Fig. 11-1, the measurement instrument 10 that measures the skin condition indicators is necessary. In the case of Embodiment 2 or 3, as shown in Fig. 11-2, the imaging device 40 that captures the image for calculating the skin condition indicators is necessary. In a case of estimating to which extent the effect can be expected for cosmetic recommendation when assuming that the application is done without actually applying the predetermined cosmetic (cosmetic to which the UV absorbing agents and/or the UV scattering agents have been added) to the skin of the user at a store or the like, it becomes possible to sequentially propose an expected level of the UV protection effect of the predetermined cosmetic and recommended cosmetics to the user by providing the measurement instrument 10 and/or the imaging device 40 in addition to the estimation system 400.

The skin condition acquisition unit 330 is a means (skin condition acquisition means) for acquiring the skin condition indicators on the basis of the measurements obtained by the measurement instrument 10 (Embodiment 1) or by using the skin image (bare skin image) captured by the imaging device 40 and the skin condition indicator mathematical model (Embodiment 2 or 3). It should be noted that in Embodiment 2 or 3, the skin condition indicators may be calculated by using the skin image (bare skin image) obtained by imaging the user and the skin condition indicator mathematical model with the information processing terminal (not shown) different from the estimation system 400 and a configuration may be made so that the calculated skin condition indicators can be acquired by the information processing terminal 300 via a network line or medium, for example. Alternatively, the skin condition indicator mathematical model may be stored in the estimation system 400, the skin image (bare skin image) captured by the imaging device 40 may be acquired, and the skin condition indicators may be calculated/acquired by the estimation system 400.

The coating film estimation unit 340 is a means (coating film estimation means) of estimating a formation state of a coating film formed on the skin by the cosmetic when assuming that a predetermined cosmetic is applied to the skin of the user by using the acquired skin condition indicators and any one of the coating film estimation mathematical models 1 to 3. The estimation method is similar to the above-mentioned one.

The evaluation unit 350 is a means of comparing and evaluating cosmetics with a published UV protection value, to which the UV absorbing agents and/or the UV scattering agents have been added, by using the formation states of the coating films formed on the skin by the cosmetics, which are estimated by the coating film estimation unit 340, and proposing a cosmetic to which the UV absorbing agents and/or the UV scattering agents have been added, which is suitable for the skin of the user. The evaluation result from the evaluation unit 350 is favorably displayed so that the user can easily get it through the display unit 360.

Displaying the evaluation result, e.g., the table shown in Fig. 9, from the evaluation unit 350 on the display unit 360 enables the user to easily get the estimation result/evaluation result.

The storage unit of the information processing terminal 300 has stored the program for executing each of the mathematical models and the estimation method. The program causes the coating film estimation unit 340 to estimate the formation state of the coating film formed on the skin by the composition by using the skin condition indicators acquired by the skin condition acquisition unit 330 and each mathematical model, causes the evaluation unit 350 to make an evaluation, and causes the display unit 360 to display the evaluation result. It should be noted that some functions may be shared by another information processing device. For example, the respective mathematical models may be stored in another apparatus, e.g., a predetermined server (not shown), and the server may be accessed from the estimation system 400 via, for example, the Internet, to cause the other apparatus to estimate the formation state of the coating film of the cosmetic by using the skin condition indicators acquired by the estimation system 400.

### <Application Program>

The estimation apparatus 200 has installed an application program (hereinafter, also referred to as this program) for causing the estimation apparatus 200 to execute the above-mentioned estimation method.

Similarly, the information processing terminal 300 has installed an application program (hereinafter, also referred to this program) for causing the information processing terminal 300 to execute the above-mentioned estimation method. In the present embodiment, the application program is an application software to estimate the formation state of the coating film of the composition in a case where the composition is applied to the skin of the user without applying the composition to the skin of the user.

This program includes skin condition acquisition processing and coating film estimation processing.

The skin condition acquisition processing is processing of acquiring skin condition indicators measured by the measurement instrument 10, skin condition indicators calculated by using the skin image (bare skin image) of the user captured by the imaging device 40 and the skin condition indicator mathematical model. It should be noted that in Embodiment 2 or 3, the skin condition indicators may be calculated by the information processing terminal (not shown) different from the estimation apparatus 200 or the information processing terminal 300 by using the skin image (bare skin image) obtained by imaging the user and the skin condition indicator mathematical model and a configuration may be made so that the calculated skin condition indicators can be acquired by the estimation apparatus 200 or the information processing terminal 300 via a network line or medium, for example. Alternatively, the skin condition indicator mathematical model may be stored in the estimation apparatus 200 or the information processing terminal 300, the skin image (bare skin image) captured by the imaging device 40 may be acquired, and the skin condition indicators may be calculated/acquired by the estimation apparatus 200 or the information processing terminal 300.

The coating film estimation processing is processing of estimating a formation state of a coating film of the cosmetic when assuming that a predetermined cosmetic is applied to the skin of the user by using the acquired skin condition indicators and any one of the coating film estimation mathematical models 1 to 3.

Moreover, this program favorably further includes evaluation processing and display processing.

The evaluation processing is processing of comparing and evaluating predetermined cosmetics with a published UV protection value, to which the UV absorbing agents and/or the UV scattering agents have been added, by using formation states of coating films formed on the skin by the cosmetics, which are estimated in the estimation processing, and proposing a cosmetic to which the UV absorbing agents and/or the UV scattering agents have been added, which is suitable for the skin of the user.

For the display processing, displaying the evaluation result of the evaluation processing, e.g., the table shown in Fig. 9, enables the user to easily get the estimation result/evaluation result.

This program can be realized as a computer program product including this program.

### <Information Processing Terminal>

The information processing terminal (not shown) may be connected to the estimation system 400 via a network. The information processing terminal may be, for example, a tablet terminal or smartphone provided at a store or a tablet terminal or smartphone possessed by the user.

The information processing terminal includes a sending means (e.g., corresponding to the input/output interface) of sending data (e.g., the skin image (bare skin image)) for determining the skin condition indicators indicating the skin characteristics of the user and a receiving means (e.g., corresponding to the input/output interface) of receiving an estimation result sent from the coating film estimation means (coating film estimation unit 340).

In this manner, with the information processing terminal including the sending means of sending the data (e.g., the skin image (bare skin image)), for example, in a case where the information processing terminal is the tablet terminal or smartphone possessed by the user, a user who hesitates to capture a facial image of the bare skin at a store captures the facial image of the bare skin of the user with the tablet terminal or smartphone possessed by the user at a home or the like and sends the facial image of the bare skin to the information processing terminal 300 of the estimation system 400 located at the store as the skin image (bare skin image) of the user. Accordingly, it is possible to estimate a formation state of the coating film when assuming that a predetermined cosmetic is applied. Moreover, with the receiving means of receiving the estimation result to the information processing terminal, it is also possible to check the estimation result with the tablet terminal or smartphone possessed by the user. It should be noted that the estimation system 400 favorably includes the evaluation unit 350 as such, and in a case where it includes the evaluation unit 350, the receiving means is favorably capable of receiving the evaluation result.

### <Display Method for Estimation Result>

On the basis of the skin condition indicators acquired by using this estimation method, the image classified in the skin type can be displayed as a skin classification image. Moreover, the predetermined composition can be displayed as a composition classification image and an evaluation based on an estimation result of a formation state of a coating film of a composition when assuming that a predetermined composition is applied to a skin of the user can be displayed by combining the skin classification image with the composition classification image.

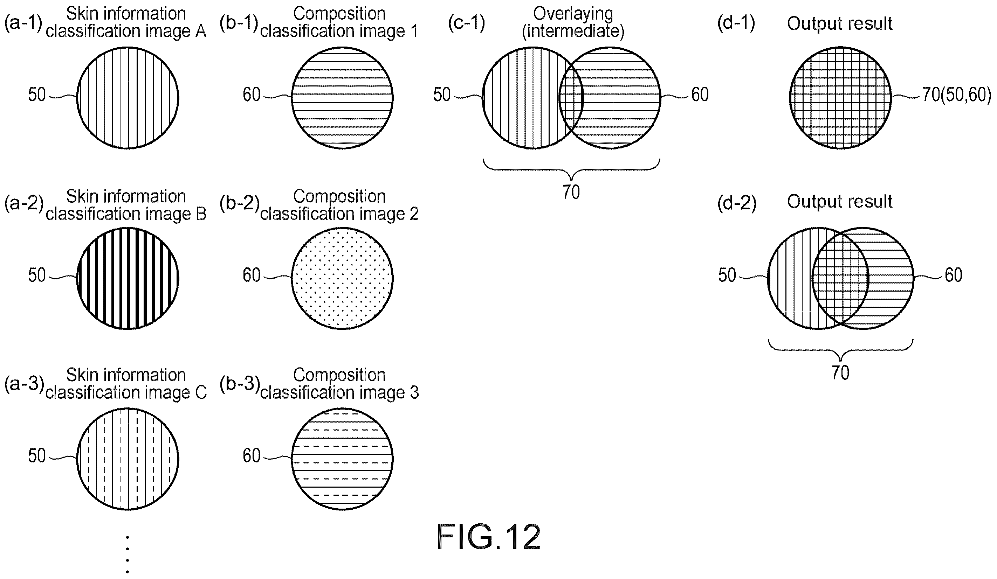

As described above, a result estimated by this estimation method or a result evaluated by using the estimated result is displayed on the display unit 160, the display unit 360, or the information processing terminal. Display formats will be described with reference to Fig. 12 (a-1) to (d-2).
Skin information classification images 50 in Fig. 12 (a-1) to (a-3) are examples of the skin information classification images 50 when classifying the skin type of the user into any one of three skin types (Skin type A, Skin type B, or Skin type C). Fig. 12 (a-1) to (a-3) show the skin information classification images 50, the skin information classification image 50 of Skin type A, the skin information classification image 50 of Skin type B, and the skin information classification image 50 of Skin type C. Moreover, Fig. 12 (b-1) to (b-3) are display examples when showing composition classification images 60 of three compositions (Composition 1, Composition 2, and Composition 3) and are the composition classification image 60 of Composition 1, the composition classification image 60 of Composition 2, and the composition classification image 60 of Composition 3. Thus, the skin information classification images 50 and the composition classification images 60 are all different images. Moreover, images that evoke the skin types may set as the skin information classification images 50. Moreover, images that evoke the compositions may set as the composition classification images 60. For example, in a case where Skin type A is a well moisturized skin type, an image designed to evoke moisture or an image designed in a color to evoke moisture. In this way, the user can easily image the skin type classified as the user's own skin type.

For example, it is possible to display compatibility of the skin type of the user with the composition by combining the skin information classification image 50 and the composition classification image 60. For example, when the skin type of the user is Skin type A, a configuration in which selecting Composition 1 reproduces a moving image in which the skin information classification image 50 in Fig. 12 (a-1) and the composition classification image 60 in Fig. 12 (b-1) are gradually overlapping each other is employed. With this configuration, it is possible to display the suitability result for Skin type A and Composition 1 in a format easy for the user to understand.

Fig. 12 (c-1) is an image showing an intermediate state when overlaying the skin information classification image 50 and the composition classification image 60.

Fig. 12 (d-1) and (d-2) are suitability images 70 each showing the estimation result, combining the skin information classification image 50 with the composition classification image 60. Fig. 12 (d-1) may be an image in a state in which the skin information classification image 50 and the composition classification image 60 perfectly overlapping each other and may be used in a case where the suitability between the skin type of the user and the composition is higher. On the other hand, Fig. 12 (d-2) may be an image in a state in which the skin information classification image 50 and the composition classification image 60 are deviated from each other and may be used in a case where the suitability between the skin type of the user and the composition is lower. In this manner, the degree of overlapping of the suitability image 70 may be used so that the user can get the compatibility between the skin type and the composition. Moreover, the estimation result only needs to display the skin information classification image 50 and the composition classification image 60 in combination regardless of how to combine (how to overlay) and how to generate the combined image. For example, the skin information classification image 50 and the composition classification image 60 are displayed side by side. For example, transparent images of the skin information classification image 50 and the composition classification image 60 are generated and displayed overlaying each other. For example, the skin information classification image 50 and the composition classification image 60 may be partially transparent images and displayed overlaying both the transparent image portions (transparent pixel portions). For example, an image in which the skin information classification image 50 and the composition classification image 60 are overlaid is generated in advance and the overlaid image generated in advance is displayed at a timing when the skin information classification image 50 and the composition classification image 60 are overlaid. In this manner, the skin information classification image 50 and the composition classification image 60 are (overlaid) displayed in combination. For example, any method can be employed, such as applying thinning processing to both the skin information classification image 50 and the composition classification image 60 (shifting the thinning locations (thinning lines) between the skin information classification image 50 and the composition classification image 60) and then overlaying them for display. Moreover, for example, in a case where the suitability between the skin type of the user and the composition is higher, the transparency of the skin information classification image 50 may be increased and the transparency of the composition classification image 60 may be decreased. On the other hand, in a case where the suitability between the skin type of the user and the composition is lower, the transparency of the skin information classification image 50 may be decreased and the transparency of the composition classification image 60 may be increased. Displaying the skin information classification image 50 and the composition classification image 60 in combination enables the user to get the suitability between the skin type of the user and the composition on the basis of the display format of the suitability image 70. Moreover, for example, in a case where the suitability between the skin type of the user and the composition is lower, the composition classification image 60 may be displayed in a smaller size than in a case where the suitability is higher. In this way, the display can be performed so that the skin information classification image 50 and the composition classification image 60 overlap each other with the same size in a case where the suitability is higher while the composition classification image 60 overlaps a portion (e.g., a center portion) of the skin information classification image 50 in a case where the suitability is lower. Therefore, it is possible to get the suitability between the skin type of the user and the composition on the basis of the display format of the suitability image 70. Moreover, for example, the brightness or saturation of the composition classification image 60 may be changed depending on the suitability between the skin type of the user and the composition and the changed composition classification image 60 may be displayed in combination with the skin information classification image 50. In this way, it is also possible to get the suitability between the skin type of the user and the composition on the basis of the suitability image 70. The above description is exemplary. Any display method and any display format may be employed for displaying the skin information classification image 50 and the composition classification image 60 in combination as long as it is possible to understand that the skin information classification image 50 and the composition classification image 60 are displayed in combination.

Moreover, a numeric value representing the suitability between the skin type of the user and the composition can also be displayed in addition to the display of the skin information classification image 50 and the composition classification image 60 in combination.

As described above, the present invention has been described showing the specific embodiment, though the present invention is not limited to the above-mentioned embodiment, and also includes aspects such as various variants and improvements as long as the object of the present invention can be achieved.

### <Modified Examples>

In the present embodiment, the coating film estimation mathematical models when the first time zone has elapsed since the predetermined cosmetic was applied to the skins of the plurality of subjects and when the second time zone has elapsed since the predetermined cosmetic was applied to the skins of the plurality of subjects are generated when estimating the first formation state and the second formation state, though not limited thereto. The coating film estimation mathematical models may be generated depending on a difference in environment, types of environment in which the first time zone and the second time zone have elapsed. For example, by generating different coating film estimation mathematical models in a case where it has elapsed under the blazing sun and in a case where it has elapsed during seaside bathing, it is possible to propose an optimal cosmetic depending on the environment where the user uses the predetermined cosmetic (cosmetic to which the UV absorbing agents and/or the UV scattering agents have been added).

In the present embodiment, the cosmetic to which the UV absorbing agents or the UV scattering agents have been added is applied as the predetermined cosmetic, though not limited thereto. Favorably, a sunscreen cosmetic containing only UV absorbing agents, only UV scattering agents, or both is applied.

In the present embodiment, the formulation type W/O is used for the cosmetic A, the formulation type W/O is used for the cosmetic B, the formulation type O/W is used for the cosmetic C, the formulation type O/W is used for the cosmetic D, and the formulation type W/O is used for the cosmetic E, though not limited thereto. Moreover, each of the cosmetics A to E used in the present embodiment is a cosmetic to which the UV absorbing agents in the range of 0 to 15 percent and the UV scattering agents in the range of 5 to 30 percent have been added, though the UV absorbing agents added to the cosmetic and/or the rate of the UV scattering agents are not limited thereto. It should be noted that a cosmetic to which the UV absorbing agents in the range of 0 to 15 percent and the UV scattering agents in the range of 5 to 30 percent have been added is favorably employed.

In the present embodiment, the cosmetic to which the UV absorbing agents and/or the UV scattering agents have been added in advance is applied as the predetermined cosmetic, though not limited thereto. By adding the UV absorbing agents or the UV scattering agents at a predetermined rate (e.g., for example, relative to the cosmetic composition, 1 mass percent or more but less than 30 mass percent, favorably 5 mass percent or more but less than 20 mass percent) to the cosmetic to which the UV absorbing agents and/or the UV scattering agents have not been added, it is possible to estimate a formation state of the coating film formed provided that it is actually applied to the skin without actually applying the cosmetic to which the UV absorbing agents and/or the UV scattering agents have not been added to the skin. In this case, either the UV absorbing agents or the UV scattering agents may be added, but it is favorable to add the UV absorbing agents. It is because adding the UV absorbing agents is less likely to affect the coating film of the cosmetic to which they are added.

As described above, in the present embodiment, the three types of estimation methods according to Embodiments 1 to 3 have been described. It is sufficient to select any one from the estimation methods depending on an estimation location or an estimation purpose. Moreover, although in Embodiment 3, the cosmetic to which the UV absorbing agents and/or the UV scattering agents have been added is applied to the skins of the plurality of subjects when generating the coating film estimation mathematical model 3, the skin of the subject is imaged without irradiating with the UV ray (it is imaged by the imaging device 40 (smartphone 40) rather than imaging it with the imaging device 30 (UV camera 30) as in Embodiments 1 and 2). Therefore, it is possible to reduce the burden on the skin of the subject when generating the coating film estimation mathematical model 3.

As described above, in the present embodiment, the formation states of the coating films when assuming that the cosmetic A and the cosmetic B are applied are estimated in Embodiment 1, the formation state of the coating film when assuming that the cosmetic C is applied in Embodiment 2, and the formation states of the coating films when assuming that the cosmetic D and the cosmetic E are applied in Embodiment 3. However, for example, it is also possible to estimate a formation state of the coating film of the cosmetic C by the method according to Embodiment 1. It is only sufficient that the coating film estimation mathematical models 1 to 3 depending on a cosmetic to be estimated are generated.

As described above, in the present embodiment, the formation state of the coating film when the cosmetic to which the UV absorbing agents or the UV scattering agents have been added is applied is estimated and the suitability to the skin of the user is evaluated on the basis of the estimation result, though not limited thereto. To which extent the function of the predetermined composition (e.g., UV protection function, anti-itch function, makeup function, tone-up function, etc.) can be exerted may be evaluated on the basis of the estimated formation state of the coating film. It is because in a case where the level of the formation state of the coating film is higher, it can be estimated that the function of the predetermined composition can be exerted at a higher level. For example, it is possible to use it as an indicator in development.

As described above, in the present embodiment, the formation state of the coating film when the cosmetic to which the UV absorbing agents or the UV scattering agents have been added is applied is estimated and the suitability of the cosmetic to the skin of the user is evaluated on the basis of the estimation result, though not limited thereto. It is also possible to evaluate the suitability between the composition and the skin of the user on the basis of the estimation result of the formation state of the coating film of the composition.

As described above, in the present embodiment, the formation state of the coating film when assuming that the cosmetic to which the UV absorbing agents or the UV scattering agents have been added is applied is estimated and the suitability to the skin of the user is evaluated by evaluating whether or not the UV protection function can be expected to be at a higher level on the basis of the estimation result, though not limited thereto. For example, when assuming that a cosmetic containing tone-up ingredients is applied, in a case where the level of the formation state of the coating film formed by applying the cosmetic is higher, it can be estimated that the tone-up ingredients can be delivered evenly to the skin. Therefore, it is also possible to evaluate the suitability between the cosmetic with the predetermined function (tone-up function) and the skin.

As described above, in the present embodiment, the "standard value" is the UV reflectance located at the center when the UV reflectances are arranged in order of the UV reflectance, which has been described in "2) Sort into two clusters in accordance with the UV reflectance," though not limited thereto. For example, the standard value may be a predetermined upper-range value (e.g., a value of the top 25 percent or less) when they are arranged in order of the UV reflectance and in a case where it is higher than the standard value, it may be evaluated that the effect claimed for the cosmetic can be expected. Moreover, the standard value may be a predetermined upper-range value (e.g., a value of the top 25 percent or less) when the coated skin overall evaluation values are arranged in order of the coated skin overall evaluation value and if it is higher than the standard value, it may be evaluated that the effect claimed for the cosmetic can be expected. Moreover, a plurality of standard values may be prepared and sorting into three or more clusters may be performed.

In the present embodiment, the first formation state and the second formation state are individually used for estimating the UV protection effects in the respective time zones, though not limited thereto. For example, an average value of the first formation state and the second formation state may be calculated and the UV protection effect that can be expected in the first time zone to the second time zone may be estimated on the basis of the average value. Alternatively, an average value may be calculated after either one of the first formation state and the second formation state is weighed (e.g., prioritizing the value of the second formation state that has elapsed longer since application, prioritizing a better formation state of the coating film, first formation state or second formation state, etc.) and the UV protection effect that can be expected in the first time zone to the second time zone may be estimated. In this manner, by estimating the UV protection effect that can be expected in the first time zone to the second time zone by using the first formation state and the second formation state, it is possible to estimate the UV protection effect that varies along with an elapse of time since application. Therefore, it is possible to provide information that the user needs in more detail.

In the present embodiment, the user is assumed to be, for example, a customer visiting a store to look for cosmetic products, though not limited thereto. For example, the user may be a monitor used during evaluations conducted at research laboratories during development of cosmetic products. The "user" in the present embodiment is a person whose skin is used for estimating the formation state of the coating film formed on the skin when the predetermined composition is applied by using the coating film estimation mathematical models 1 to 3 without actually applying the predetermined composition. The "subject" is a person whose skin is used as training data when generating each mathematical model.

### (Additional Test Example 1)

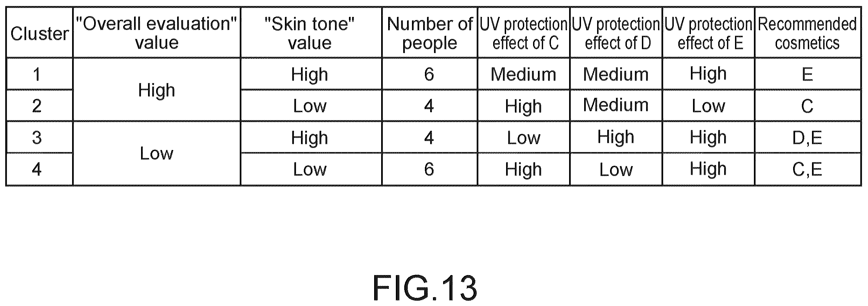

Fig. 13 is a diagram showing results of comparison tests of the UV protection effects of the cosmetics C to E. As shown in the figure, regarding the "overall evaluation" and "skin tone" determined using the above-mentioned skin condition indicator mathematical model, four clusters classified into two clusters were generated using a median value as a threshold and the UV protection effects of the cosmetic C (Embodiment 2), the cosmetic D (Embodiment 3), and the cosmetic E (Embodiment 3) were compared for each cluster.

As a result, it was found that as the UV protection effect, the cosmetic E was recommended for a cluster 1 whose overall evaluation was higher and whose skin tone value was also higher, the cosmetic C was recommended for a cluster 2 whose overall evaluation was higher and whose skin tone value was lower, the cosmetics D and E were recommended for a cluster 3 whose overall evaluation was lower and whose skin tone value was higher, and the cosmetics C and E were recommended for a cluster 4 whose overall evaluation was lower and whose skin tone value was also higher.

In this manner, not only in a case of using the skin condition indicators of the seven items in the above-mentioned embodiment as explanatory variables, but also in a case of using the two items, "overall evaluation" and "skin tone," as explanatory variables for classifying into the four skin types, it is possible to recommend a cosmetic whose UV protection effect is higher to that skin type to the user.

### (Additional Test Example 2)

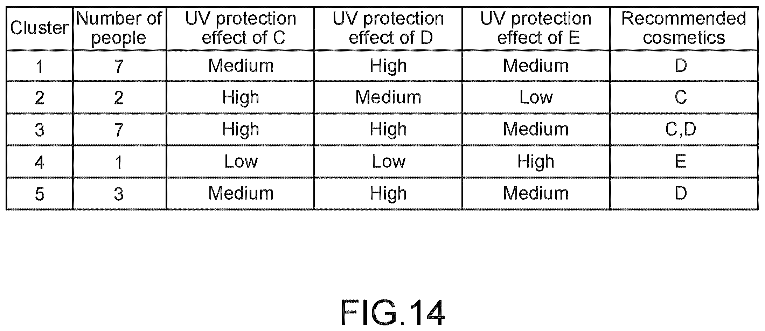

Fig. 14 is a diagram showing results of other comparison tests of the UV protection effects of the cosmetics C to E. Participants of the tests were classified into five clusters by a non-hierarchical cluster method (k-means method) using the seven items, "overall evaluation," "skin tone," "makeup intensity," "skin age," "masculinity/femininity," "degree of makeup smudging," and the "powderiness," determined using the above-mentioned skin condition indicator mathematical model and the UV protection effects of the cosmetic C (Embodiment 2), the cosmetic D (Embodiment 3), and the cosmetic E (Embodiment 3) were compared for each cluster.

As a result, it was found that the UV protection effects of the cosmetics C to E were different for each of the clusters 1 to 5. It was found that as the UV protection effect, the cosmetic D was recommended for the cluster 1, the cosmetic C was recommended for the cluster 2, the cosmetics C and D were recommended for the cluster 3, the cosmetic E was recommended for the cluster 4, and the cosmetic D was recommended for the cluster 5.

In this manner, also in a case of classifying into the five skin types by using the skin condition indicators of the seven items in the above-mentioned embodiment as explanatory variables, it is possible to recommend a cosmetic whose UV protection effect is higher to that skin type to the user.

### (Cosmetic Proposing Processing Depending on Skin Type to User)

The above-mentioned embodiment shows the example in which the user's information processing terminal is caused to display the image classified in the skin type as the skin classification image on the basis of the estimated skin condition indicators and the user's information processing terminal is caused to display the predetermined composition as the composition classification image, and the evaluation based on the estimation result of the formation state of the coating film of the composition when assuming that the predetermined composition is applied to the skin of the user is displayed by combining the skin classification image with the composition classification image.

At this time, the estimation apparatus 200 or the estimation system 400 may cause the user's information processing terminal to display the information indicating the characteristics of the classified skin type and also causes the user's information processing terminal to display a score indicating compatibility (UV protection effect) with the skin type of the user for each cosmetic corresponding to the skin type.

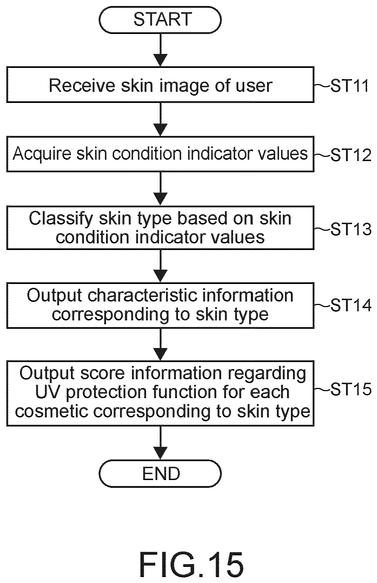

Fig. 15 is a flowchart showing a flow of presentation processing of the skin type characteristic information and the cosmetic score information based on the skin image by the estimation apparatus 200 or the estimation system 400. Hereinafter, for the sake of convenience, the estimation apparatus 200 is described as a subject for the operation, though this operation is executed by cooperation of hardware, such as CPU, communication unit, and storage unit of the estimation apparatus 200 or the estimation system, with software (programs) stored in the storage unit.

As shown in the figure, the estimation apparatus 200 receives a skin image of a bare face of the user, which has been captured by the information processing terminal, from the user's information processing terminal (Step 11).

Subsequently, the estimation apparatus 200 acquires skin condition indicator values on the basis of the received skin image and the above-mentioned skin condition indicator mathematical model (Step 12).

Subsequently, the estimation apparatus 200 classifies the user's skin type into any one skin type on the basis of the acquired skin condition indicator values (Step 13).

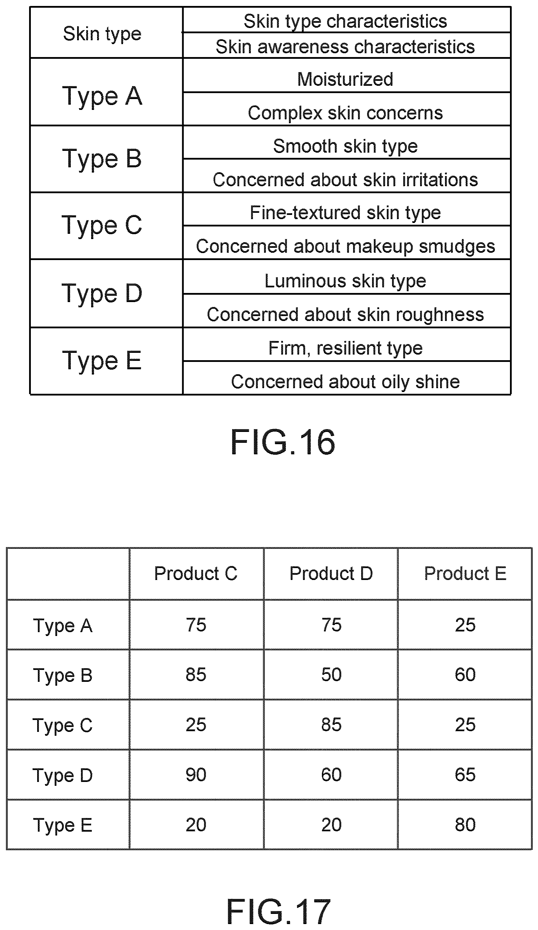

When generating classification information of the skin type, the estimation apparatus 200 or the estimation system 400 generates and stores characteristic information for describing characteristics of each skin type, for example, as shown in Fig. 16. The characteristic information is generated by evaluating each skin type on the basis of the skin condition indicator values and the subject's awareness of the skin type.

The estimation apparatus 200 or the estimation system 400 also generates and stores score information indicating scores of UV protection effects of multiple types of cosmetics on the basis of formation states of the coating film for each skin type.

For example, as shown in Fig. 17, the score information is generated by creating a matrix of skin types and cosmetics. The score information is calculated as a numeric value by using, for example, the UV reflectance of the coating film and the overall evaluation value immediately after application or after a period of time has elapsed since application. When comparing cosmetics within the same skin type, a higher score indicates greater UV protection effect.

The score information is not limited to those represented as a value of 0 to 100 as described above. For example, the score information may be represented as a value of 0 to 10. For example, 5-scale evaluation information, such as A, B, C, D, or E (or the number of stars, etc.). Any evaluation information may be employed.

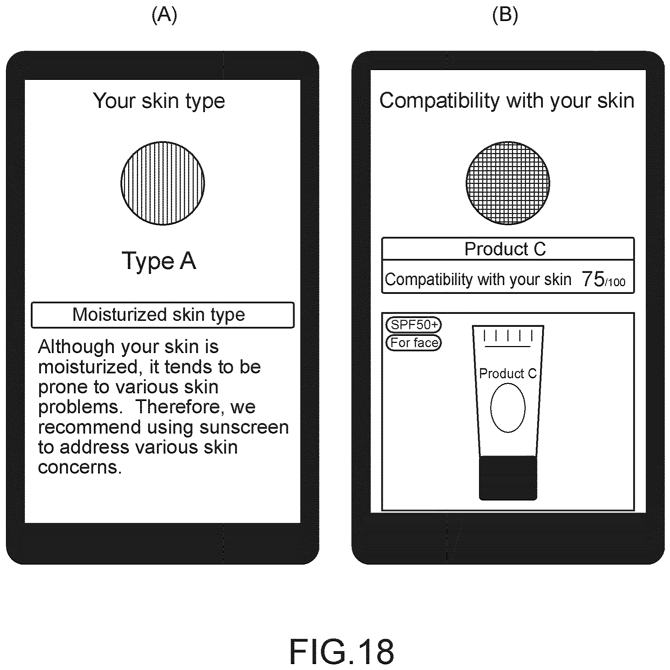

Subsequently, the estimation apparatus 200 causes the user's information processing terminal to display characteristic information corresponding to the skin type out of the above-mentioned stored characteristic information with the information regarding the classified skin type of the user as shown in Fig. 18 (A) (Step 14). In the example in the figure, the user's skin type is classified into Skin type A and the characteristic information (Moisturized skin type) corresponding to it is shown with the information regarding Skin type A.

Then, as shown in Fig. 18 (B), for example, when the user scrolls/taps on the displayed characteristic information or when a predetermined time has elapsed since the display start, the estimation apparatus 200 causes the user's information processing terminal to display the information regarding the cosmetic whose UV protection effect is higher and its score information for the skin type (Step 15).

Here, regarding the cosmetic and score to be displayed, at least a cosmetic with the highest score is displayed for the classified skin type. Information on a plurality of cosmetics at upper-levels (Ranks 1 to 3, etc.) and their scores may be displayed.

In this manner, it is possible to classify the user into any one of multiple skin types on the basis of the skin condition indicators acquired on the basis of the skin image of the user and the skin condition indicator mathematical model, and present characteristic information of that skin type and information regarding a cosmetic with a high UV protection effect for that skin type to the user so that the user can get it.

### Reference Signs List

- 10: measurement instrument
- 15: skin
- 20: composition
- 30: imaging device (UV camera)
- 40: imaging device (smartphone)
- 50: skin information classification image
- 60: composition classification image
- 70: suitability image
- 130: skin condition acquisition unit
- 140: coating film estimation unit
- 150: evaluation unit
- 160: display unit
- 200: estimation apparatus
- 330: skin condition acquisition unit
- 340: coating film estimation unit
- 350: evaluation unit
- 360: display unit
- 400: estimation system

## Claims

1. An estimation method, comprising:
acquiring skin condition indicators indicating skin characteristics of a user; and
estimating, by using the acquired skin condition indicators and a mathematical model indicating a relationship between the skin condition indicators and formation states of a coating film of a composition when a predetermined composition is applied to a skin of the user, a formation state of a coating film of a composition when assuming that a predetermined composition is applied to the skin of the user and estimating a UV protection function on a basis of a result thereof.

2. The estimation method according to claim 1, **characterized in that**
the skin condition indicators include evaluation items based on physical property values indicating skin conditions measured by a predetermined measurement instrument.

3. The estimation method according to claim 2, **characterized in that**
the evaluation items based on the physical property value include at least one of a stratum corneum water content, a transepidermal water loss, skin viscoelasticity, skin elasticity, or a contact angle.

4. The estimation method according to claim 1, **characterized in that**
the skin condition indicators include evaluation items, which are acquired by using a skin image obtained by imaging the skin of the user and a skin condition indicator mathematical model indicating a relationship between the skin image and the skin condition indicators.

5. The estimation method according to any one of claims 1 to 4, **characterized in that**
the predetermined composition is a cosmetic to which at least either ingredients that absorb UV rays or ingredients that scatter UV rays have been added, and
the estimation method is an estimation method of estimating the formation state of the coating film of the cosmetic on a basis of the skin condition indicators acquired from the user and the mathematical model and estimating a UV protection function on a basis of a result thereof, the method comprising:
acquiring each of skin condition indicators of a plurality of subjects; and
acquiring each of UV reflectances of coating films of the cosmetic from UV skin images, which are obtained by imaging skins of the plurality of subjects to which the cosmetic has been applied under UV irradiation, **characterized in that**
the mathematical model is generated by performing machine learning on a basis of the acquired skin condition indicators of the plurality of subjects and the acquired UV reflectances of the coating films of the cosmetic of the plurality of subjects.

6. The estimation method according to any one of claims 1 to 4, **characterized in that**
the predetermined composition is a cosmetic to which at least either ingredients that absorb UV rays or ingredients that scatter UV rays have been added, and
the estimation method is an estimation method of estimating the formation state of the coating film of the cosmetic on a basis of the skin condition indicators acquired from the user and the mathematical model and estimating a UV protection function on a basis of a result thereof, the method comprising:
acquiring each of skin condition indicators of a plurality of subjects; and
acquiring each of coated skin overall evaluations indicating conditions of skins of the plurality of subjects to which the cosmetic has been applied, **characterized in that**
the coated skin overall evaluations are evaluation items acquired by using coated skin images obtained by imaging the skins of the subjects to which the cosmetic has been applied and a skin condition indicator mathematical model indicating a relationship between the coated skin images and the coated skin overall evaluations, and
the mathematical model is generated by performing machine learning on a basis of the acquired skin condition indicators of the plurality of subjects and the acquired coated skin overall evaluations of the plurality of subjects.

7. The estimation method according to any one of claims 1 to 6, **characterized by** further comprising evaluating a function of the composition on a basis of the estimated formation state of the coating film of the composition.

8. The estimation method according to any one of claims 1 to 7, wherein
the predetermined composition is a cosmetic to which at least either ingredients that absorb UV rays or ingredients that scatter UV rays have been added, and
the cosmetic has a published UV protection value published in advance,
the method further comprising estimating and evaluating the UV protection function as a predetermined function on a basis of the estimated formation state of the coating film of the cosmetic and the published UV protection value.

9. The estimation method according to any one of claims 1 to 7, wherein
the predetermined composition is a cosmetic to which at least either ingredients that absorb UV rays or ingredients that scatter UV rays have been added, and
the cosmetic has a predetermined function,
the method further comprising evaluating suitability of the cosmetic to the skin of the user by using the estimated formation state of the coating film of the cosmetic.

10. The estimation method according to any one of claims 1 to 9, **characterized by** further comprising estimating at least one of a first formation state that is a formation state of the coating film of the composition in a first time zone after application in which the predetermined composition is applied or a second formation state that is a formation state of the coating film of the composition in a second time zone later than the first time zone.

11. The estimation method according to any one of claims 1 to 10, **characterized by** further comprising:
classifying the skin of the user as any one of multiple skin types on a basis of the skin condition indicators;
determining a skin information classification image indicating the skin type for each of the multiple skin types in advance;
determining a composition classification image indicating the predetermined composition in advance; and
displaying the estimation result with the skin information classification image and the composition classification image.

12. The estimation method according to claim 11, **characterized in that** the estimation result is displayed, combining the skin information classification image with the composition classification image.

13. The estimation method according to claim 4, further comprising:
storing characteristic information indicating characteristics of multiple skin types and evaluation information indicating evaluations related to UV protection functions of one or more cosmetics based on formation states of the coating film for each of the multiple skin types;
classifying the skin of the user as any one of the multiple skin types on a basis of the skin condition indicators acquired on a basis of the skin image and the skin condition indicator mathematical model;
outputting the characteristic information corresponding to the classified skin type; and
outputting the evaluation information for each of the cosmetics corresponding to the classified skin type.

14. An estimation apparatus, comprising:
a storage means of storing skin condition indicators indicating skin characteristics of a user and information indicating the correlation between the skin condition indicators and formation states of a coating film of a composition when a predetermined composition is applied to a skin of the user;
a skin condition acquisition means of acquiring the skin condition indicators; and
a coating film estimation means of referring to the storage means, estimating a formation state of a coating film of a composition when assuming that a predetermined composition is applied to the skin of the user by using the acquired skin condition indicators, and estimating a UV protection function on a basis of a result thereof.

15. An application program that operates an estimation apparatus that estimates a formation state of a coating film of a composition when assuming that a predetermined composition is applied to a skin of a user and estimates a UV protection function on a basis of a result thereof, comprising:
skin condition acquisition processing of acquiring skin condition indicators indicating skin characteristics of the user; and
coating film estimation processing of estimating the formation state of the coating film of the composition when assuming that the predetermined composition is applied to the skin of the user by using the acquired skin condition indicators and estimating a UV protection function on a basis of a result thereof.

16. An estimation system, comprising:
a storage means of storing skin condition indicators indicating skin characteristics of a user and information indicating the correlation between the skin condition indicators and formation states of a coating film of a composition when a predetermined composition is applied to a skin of the user;
a skin condition acquisition means of acquiring the skin condition indicators; and
a coating film estimation means of referring to the storage means, estimating a formation state of a coating film of a composition when assuming that a predetermined composition is applied to the skin of the user by using the acquired skin condition indicators, and estimating a UV protection function on a basis of a result thereof.

17. An information processing terminal for use in an estimation system connected to an estimation apparatus according to claim 14 via a network, the estimation system including:
a sending means of sending data for determining skin condition indicators indicating skin characteristics of a user; and
a receiving means of receiving an estimation result sent from the coating film estimation means.

18. A storage medium that has stored an application program that operates an estimation apparatus that estimates a formation state of a coating film of a composition when assuming that a predetermined composition is applied to a skin of a user and estimates a UV protection function on a basis of a result thereof, the application program including
skin condition acquisition processing of acquiring skin condition indicators indicating skin characteristics of the user, and
coating film estimation processing of estimating the formation state of the coating film of the composition when assuming that the predetermined composition is applied to the skin of the user by using the acquired skin condition indicators and estimating a UV protection function on a basis of a result thereof.
